# EUROPEAN PATENT APPLICATION

(11) **EP 3 818 989 A1**
(43) Date of publication of application: **12.05.2021**
(21) Application number: 20162831.0
(22) Date of filing: 12.03.2020
(51) Int. Cl.: A61K 39/12, A61K 39/215, A61P 31/14

(54) **PEPTIDES AND OLIGONUCLEOTIDES FOR A SARS-COV-2 VACCINE**

(71) Applicant: Universität Greifswald, 17489 Greifswald (DE)
(72) Inventor: Lucchese, Guglielmo, 17475 Greifswald (DE); Flöel, Agnes, 17475 Greifswald (DE)
(74) Representative: Moré, Solveig Helga

(57) **Abstract**

The invention relates to peptides derived from the spike glycoprotein of Severe Acute Respiratory Syndrome Coronavirus 2 (SARS-CoV-2 or 2019-nCoV) and nucleic acids encoding the same. These, and compositions comprising them can advantageously be used for vaccination against coronaviruses, in particular, as immunogenic antigens in vaccine formulations to fight SARS-CoV-2 and/or other coronaviruses. Such vaccines may also protect against further infectious agents, e.g., SARS-CoV and/or influenza.

SEQUENCE LISTING

<110> Universität Greifswald

<120> Peptides and oligonucleotides for a SARS-CoV-2 vaccine

<130> 12600

<160> 140

<170> BiSSAP 1.3.6

<210> 1
<211> 9
<212> PRT
<213> SARS coronavirus


<400> 1
Phe Ala Met Gln Met Ala Tyr Arg Phe
1               5

<210> 2
<211> 7
<212> PRT
<213> SARS coronavirus


<400> 2
Phe Lys Cys Tyr Gly Val Ser
1               5

<210> 3
<211> 7
<212> PRT
<213> SARS coronavirus


<400> 3
Ile Lys Trp Pro Trp Tyr Ile
1               5

<210> 4
<211> 8
<212> PRT
<213> SARS coronavirus


<400> 4
Arg Gly Val Tyr Tyr Pro Asp Lys
1               5

<210> 5
<211> 7
<212> PRT
<213> SARS coronavirus


<400> 5
Asn Val Thr Trp Phe His Ala
1               5

<210> 6

<211> 5
<212> PRT
<213> SARS coronavirus

<400> 6
Phe His Ala Ile His
1               5

<210> 7
<211> 5
<212> PRT
<213> SARS coronavirus

<400> 7
Pro Phe Asn Asp Gly
1               5

<210> 8
<211> 6
<212> PRT
<213> SARS coronavirus

<400> 8
Ile Arg Gly Trp Ile Phe
1               5

<210> 9
<211> 5
<212> PRT
<213> SARS coronavirus

<400> 9
Ile Phe Gly Thr Thr
1               5

<210> 10
<211> 7
<212> PRT
<213> SARS coronavirus

<400> 10
Val Cys Glu Phe Gln Phe Cys
1               5

<210> 11
<211> 5
<212> PRT
<213> SARS coronavirus

<400> 11
Cys Asn Asp Pro Phe
1               5

<210> 12
<211> 5
<212> PRT
<213> SARS coronavirus

<400> 12
Val Tyr Tyr His Lys
1               5

<210> 13
<211> 5
<212> PRT
<213> SARS coronavirus

<400> 13
Asn Asn Lys Ser Trp
1               5

<210> 14
<211> 4
<212> PRT
<213> SARS coronavirus

<400> 14
Lys Ser Trp Met
1

<210> 15
<211> 6
<212> PRT
<213> SARS coronavirus

<400> 15
Trp Met Glu Ser Glu Phe
1               5

<210> 16
<211> 5
<212> PRT
<213> SARS coronavirus

<400> 16
Tyr Ser Ser Ala Asn
1               5

<210> 17
<211> 5
<212> PRT
<213> SARS coronavirus

<400> 17
Cys Thr Phe Glu Tyr
1               5

<210> 18
<211> 5
<212> PRT
<213> SARS coronavirus

<400> 18

Gly Asn Phe Lys Asn
1               5

<210> 19
<211> 5
<212> PRT
<213> SARS coronavirus


<400> 19
Gly Tyr Phe Lys Ile
1               5

<210> 20
<211> 6
<212> PRT
<213> SARS coronavirus


<400> 20
Ile Tyr Ser Lys His Thr
1               5

<210> 21
<211> 5
<212> PRT
<213> SARS coronavirus


<400> 21
Pro Ile Gly Ile Asn
1               5

<210> 22
<211> 5
<212> PRT
<213> SARS coronavirus


<400> 22
Gly Trp Thr Ala Gly
1               5

<210> 23
<211> 5
<212> PRT
<213> SARS coronavirus


<400> 23
Ala Tyr Tyr Val Gly
1               5

<210> 24
<211> 5
<212> PRT
<213> SARS coronavirus


<400> 24
Asn Glu Asn Gly Thr
1               5

<210> 25
<211> 5
<212> PRT
<213> SARS coronavirus

<400> 25
Ser Glu Thr Lys Cys
1               5

<210> 26
<211> 5
<212> PRT
<213> SARS coronavirus

<400> 26
Gly Ile Tyr Gln Thr
1               5

<210> 27
<211> 6
<212> PRT
<213> SARS coronavirus

<400> 27
Val Tyr Ala Trp Asn Arg
1               5

<210> 28
<211> 5
<212> PRT
<213> SARS coronavirus

<400> 28
Cys Val Ala Asp Tyr
1               5

<210> 29
<211> 5
<212> PRT
<213> SARS coronavirus

<400> 29
Ser Thr Phe Lys Cys
1               5

<210> 30
<211> 5
<212> PRT
<213> SARS coronavirus

<400> 30
Thr Asn Val Tyr Ala
1               5

<210> 31
<211> 5
<212> PRT

<213> SARS coronavirus

<400> 31
Ile Ala Asp Tyr Asn
1               5

<210> 32
<211> 6
<212> PRT
<213> SARS coronavirus

<400> 32
Asp Tyr Asn Tyr Lys Leu
1               5

<210> 33
<211> 5
<212> PRT
<213> SARS coronavirus

<400> 33
Val Ile Ala Trp Asn
1               5

<210> 34
<211> 6
<212> PRT
<213> SARS coronavirus

<400> 34
Ala Trp Asn Ser Asn Asn
1               5

<210> 35
<211> 5
<212> PRT
<213> SARS coronavirus

<400> 35
Ser Thr Pro Cys Asn
1               5

<210> 36
<211> 5
<212> PRT
<213> SARS coronavirus

<400> 36
Pro Cys Asn Gly Val
1               5

<210> 37
<211> 6
<212> PRT
<213> SARS coronavirus

<400> 37
Gly Phe Asn Cys Tyr Phe
1               5

<210> 38
<211> 5
<212> PRT
<213> SARS coronavirus

<400> 38
Gln Ser Tyr Gly Phe
1               5

<210> 39
<211> 5
<212> PRT
<213> SARS coronavirus

<400> 39
Val Lys Asn Lys Cys
1               5

<210> 40
<211> 5
<212> PRT
<213> SARS coronavirus

<400> 40
Asn Lys Cys Val Asn
1               5

<210> 41
<211> 5
<212> PRT
<213> SARS coronavirus

<400> 41
Cys Val Asn Phe Asn
1               5

<210> 42
<211> 5
<212> PRT
<213> SARS coronavirus

<400> 42
Cys Thr Glu Val Pro
1               5

<210> 43
<211> 5
<212> PRT
<213> SARS coronavirus

<400> 43
Ile Gly Ala Glu His
1               5

<210> 44
<211> 6
<212> PRT
<213> SARS coronavirus

<400> 44
Tyr Gln Thr Gln Thr Asn
1               5

<210> 45
<211> 6
<212> PRT
<213> SARS coronavirus

<400> 45
Ile Ala Tyr Thr Met Ser
1               5

<210> 46
<211> 5
<212> PRT
<213> SARS coronavirus

<400> 46
Thr Ser Val Asp Cys
1               5

<210> 47
<211> 5
<212> PRT
<213> SARS coronavirus

<400> 47
Asp Cys Thr Met Tyr
1               5

<210> 48
<211> 6
<212> PRT
<213> SARS coronavirus

<400> 48
Thr Met Tyr Ile Cys Gly
1               5

<210> 49
<211> 5
<212> PRT
<213> SARS coronavirus

<400> 49
Asp Ser Thr Glu Cys
1               5

<210> 50
<211> 5

<212> PRT
<213> SARS coronavirus

<400> 50
Phe Cys Thr Gln Leu
1               5

<210> 51
<211> 5
<212> PRT
<213> SARS coronavirus

<400> 51
Pro Ile Lys Asp Phe
1               5

<210> 52
<211> 6
<212> PRT
<213> SARS coronavirus

<400> 52
Gln Tyr Gly Asp Cys Leu
1               5

<210> 53
<211> 5
<212> PRT
<213> SARS coronavirus

<400> 53
Gly Asp Cys Leu Gly
1               5

<210> 54
<211> 8
<212> PRT
<213> SARS coronavirus

<400> 54
Asp Leu Ile Cys Ala Gln Lys Phe
1               5

<210> 55
<211> 5
<212> PRT
<213> SARS coronavirus

<400> 55
Met Ile Ala Gln Tyr
1               5

<210> 56
<211> 5
<212> PRT
<213> SARS coronavirus

<400> 56
Ser Gly Trp Thr Phe
1               5

<210> 57
<211> 5
<212> PRT
<213> SARS coronavirus

<400> 57
Trp Thr Phe Gly Ala
1               5

<210> 58
<211> 5
<212> PRT
<213> SARS coronavirus

<400> 58
Phe Ala Met Gln Met
1               5

<210> 59
<211> 7
<212> PRT
<213> SARS coronavirus

<400> 59
Met Gln Met Ala Tyr Arg Phe
1               5

<210> 60
<211> 5
<212> PRT
<213> SARS coronavirus

<400> 60
Arg Phe Asn Gly Ile
1               5

<210> 61
<211> 5
<212> PRT
<213> SARS coronavirus

<400> 61
Met Ser Glu Cys Val
1               5

<210> 62
<211> 6
<212> PRT
<213> SARS coronavirus

<400> 62
Gly Tyr His Leu Met Ser

1                   5

<210> 63
<211> 5
<212> PRT
<213> SARS coronavirus


<400> 63
Lys Asn Phe Thr Thr
1                   5

<210> 64
<211> 5
<212> PRT
<213> SARS coronavirus


<400> 64
Pro Ala Ile Cys His
1                   5

<210> 65
<211> 9
<212> PRT
<213> SARS coronavirus


<400> 65
Asn Gly Thr His Trp Phe Val Thr Gln
1                   5

<210> 66
<211> 5
<212> PRT
<213> SARS coronavirus


<400> 66
Thr Gln Arg Asn Phe
1                   5

<210> 67
<211> 5
<212> PRT
<213> SARS coronavirus


<400> 67
Asn Phe Tyr Glu Pro
1                   5

<210> 68
<211> 5
<212> PRT
<213> SARS coronavirus


<400> 68
Ile Gly Ile Val Asn
1                   5

<210> 69

<211> 5
<212> PRT
<213> SARS coronavirus

<400> 69
Asn Thr Val Tyr Asp
1               5

<210> 70
<211> 6
<212> PRT
<213> SARS coronavirus

<400> 70
Tyr Ile Trp Leu Gly Phe
1               5

<210> 71
<211> 5
<212> PRT
<213> SARS coronavirus

<400> 71
Ile Ala Ile Val Met
1               5

<210> 72
<211> 6
<212> PRT
<213> SARS coronavirus

<400> 72
Leu Cys Cys Met Thr Ser
1               5

<210> 73
<211> 5
<212> PRT
<213> SARS coronavirus

<400> 73
Met Thr Ser Cys Cys
1               5

<210> 74
<211> 5
<212> PRT
<213> SARS coronavirus

<400> 74
Cys Cys Lys Phe Asp
1               5

<210> 75
<211> 17
<212> PRT
<213> Artificial Sequence

<220>
<223> epitope comprising SARS-CoV-2 sequence

<400> 75
Ala Ala Leu Val Ser Gly Thr Ala Thr Ala Gly Trp Thr Phe Gly Ala
1               5                   10                  15
Gly


<210> 76
<211> 17
<212> PRT
<213> Artificial Sequence


<220>
<223> epitope comprising SARS-CoV-2 sequence

<400> 76
Ala Ala Thr Lys Met Ser Glu Cys Val Leu Gly Gln Ser Lys Arg Val
1               5                   10                  15
Asp


<210> 77
<211> 17
<212> PRT
<213> Artificial Sequence


<220>
<223> epitope comprising SARS-CoV-2 sequence

<400> 77
Ala Gly Cys Leu Ile Gly Ala Glu His Val Asp Thr Ser Tyr Glu Cys
1               5                   10                  15
Asp


<210> 78
<211> 8
<212> PRT
<213> Artificial Sequence


<220>
<223> epitope comprising SARS-CoV-2 sequence

<400> 78
Ala Met Gln Met Ala Tyr Arg Phe
1               5

<210> 79
<211> 23
<212> PRT
<213> Artificial Sequence


<220>
<223> epitope comprising SARS-CoV-2 sequence

<400> 79
Cys Ala Asn Leu Leu Leu Gln Tyr Gly Ser Phe Cys Thr Gln Leu Asn
1               5                   10                  15
Arg Ala Leu Ser Gly Ile Ala
            20

<210> 80
<211> 42
<212> PRT
<213> Artificial Sequence

<220>
<223> epitope comprising SARS-CoV-2 sequence

<400> 80
Cys Gly Pro Lys Leu Ser Thr Asp Leu Ile Lys Asn Gln Cys Val Asn
1               5                   10                  15
Phe Asn Phe Asn Gly Leu Thr Gly Thr Gly Val Leu Thr Pro Ser Ser
            20                  25                  30
Lys Arg Phe Gln Pro Phe Gln Gln Phe Gly
        35                  40

<210> 81
<211> 50
<212> PRT
<213> Artificial Sequence

<220>
<223> epitope comprising SARS-CoV-2 sequence

<400> 81
Cys Gly Pro Lys Leu Ser Thr Asp Leu Ile Lys Asn Gln Cys Val Asn
1               5                   10                  15
Phe Asn Phe Asn Gly Leu Thr Gly Thr Gly Val Leu Thr Pro Ser Ser
            20                  25                  30
Lys Arg Phe Gln Pro Phe Gln Gln Phe Gly Arg Asp Val Ser Asp Phe
        35                  40                  45
Thr Asp
    50

<210> 82
<211> 35
<212> PRT
<213> Artificial Sequence

<220>
<223> epitope comprising SARS-CoV-2 sequence

<400> 82
Cys Ser Gln Asn Pro Leu Ala Glu Leu Lys Cys Ser Val Lys Ser Phe
1               5                   10                  15
Glu Ile Asp Lys Gly Ile Tyr Gln Thr Ser Asn Phe Arg Val Val Pro
            20                  25                  30
Ser Gly Asp
        35

<210> 83
<211> 30
<212> PRT
<213> Artificial Sequence

<220>
<223> epitope comprising SARS-CoV-2 sequence

<400> 83
Cys Thr Thr Phe Asp Asp Val Gln Ala Pro Asn Tyr Thr Gln His Thr
1               5                   10                  15
Ser Ser Met Arg Gly Val Tyr Tyr Pro Asp Glu Ile Phe Arg
            20                  25                  30

<210> 84
<211> 17
<212> PRT
<213> Artificial Sequence

<220>
<223> epitope comprising SARS-CoV-2 sequence

<400> 84
Cys Tyr Gly Val Ser Ala Thr Lys Leu Asn Asp Leu Cys Phe Ser Asn
1               5                   10                  15
Val

<210> 85
<211> 17
<212> PRT
<213> Artificial Sequence

<220>
<223> epitope comprising SARS-CoV-2 sequence

<400> 85
Asp Phe Cys Gly Lys Gly Tyr His Leu Met Ser Phe Pro Gln Ala Ala
1               5                   10                  15
Pro

<210> 86
<211> 17
<212> PRT
<213> Artificial Sequence

<220>
<223> epitope comprising SARS-CoV-2 sequence

<400> 86
Glu Ile Asp Lys Gly Ile Tyr Gln Thr Ser Asn Phe Arg Val Val Pro
1               5                   10                  15
Ser

<210> 87
<211> 17
<212> PRT
<213> Artificial Sequence

<220>

<223> epitope comprising SARS-CoV-2 sequence

<400> 87
Phe Phe Ser Thr Phe Lys Cys Tyr Gly Val Ser Ala Thr Lys Leu Asn
1               5                   10                  15
Asp

<210> 88
<211> 17
<212> PRT
<213> Artificial Sequence

<220>
<223> epitope comprising SARS-CoV-2 sequence

<400> 88
Phe Val Phe Asn Gly Thr Ser Trp Phe Ile Thr Gln Arg Asn Phe Phe
1               5                   10                  15
Ser

<210> 89
<211> 17
<212> PRT
<213> Artificial Sequence

<220>
<223> epitope comprising SARS-CoV-2 sequence

<400> 89
Gly Ala Ala Leu Gln Ile Pro Phe Ala Met Gln Met Ala Tyr Arg Phe
1               5                   10                  15
Asn

<210> 90
<211> 20
<212> PRT
<213> Artificial Sequence

<220>
<223> epitope comprising SARS-CoV-2 sequence

<400> 90
Gly Asn Leu Ile Ala Pro Arg Gly Tyr Phe Lys Ile Arg Ser Gly Lys
1               5                   10                  15
Ser Ser Ile Met
            20

<210> 91
<211> 8
<212> PRT
<213> Artificial Sequence

<220>
<223> epitope comprising SARS-CoV-2 sequence

<400> 91

Gly Ser Phe Cys Thr Gln Leu Asn
1               5

<210> 92
<211> 17
<212> PRT
<213> Artificial Sequence

<220>
<223> epitope comprising SARS-CoV-2 sequence

<400> 92
His Thr Ser Ser Met Arg Gly Val Tyr Tyr Pro Asp Glu Ile Phe Arg
1               5                   10                  15
Ser

<210> 93
<211> 17
<212> PRT
<213> Artificial Sequence

<220>
<223> epitope comprising SARS-CoV-2 sequence

<400> 93
Ile Ala Asp Tyr Asn Tyr Lys Leu Pro Asp Asp Phe Met Gly Cys Val
1               5                   10                  15
Leu

<210> 94
<211> 17
<212> PRT
<213> Artificial Sequence

<220>
<223> epitope comprising SARS-CoV-2 sequence

<400> 94
Ile Ala Gly Leu Ile Ala Ile Val Met Val Thr Ile Leu Leu Cys Cys
1               5                   10                  15
Met

<210> 95
<211> 17
<212> PRT
<213> Artificial Sequence

<220>
<223> epitope comprising SARS-CoV-2 sequence

<400> 95
Ile Ala Pro Gly Gln Thr Gly Val Ile Ala Asp Tyr Asn Tyr Lys Leu
1               5                   10                  15
Pro

<210> 96
<211> 32
<212> PRT
<213> Artificial Sequence

<220>
<223> epitope comprising SARS-CoV-2 sequence

<400> 96
Ile Ala Pro Arg Gly Tyr Phe Lys Ile Arg Asn Gly Lys Ser Ser Ile
1               5                   10                  15
Met Arg Ser Asp Ala Pro Ile Gly Thr Cys Ser Ser Glu Cys Ile Thr
            20                  25                  30

<210> 97
<211> 31
<212> PRT
<213> Artificial Sequence

<220>
<223> epitope comprising SARS-CoV-2 sequence

<400> 97
Ile Tyr Trp Thr Ile Val Lys Pro Gly Asp Ile Leu Leu Ile Asn Ser
1               5                   10                  15
Thr Gly Asn Leu Ile Ala Pro Arg Gly Tyr Phe Lys Ile Arg Asn
            20                  25                  30

<210> 98
<211> 8
<212> PRT
<213> Artificial Sequence

<220>
<223> epitope comprising SARS-CoV-2 sequence

<400> 98
Lys Gly Ile Tyr Gln Thr Ser Asn
1               5

<210> 99
<211> 20
<212> PRT
<213> Artificial Sequence

<220>
<223> epitope comprising SARS-CoV-2 sequence

<400> 99
Lys Gly Ile Tyr Gln Thr Ser Asn Phe Arg Val Val Pro Ser Gly Asp
1               5                   10                  15
Val Val Arg Phe
            20

<210> 100
<211> 17
<212> PRT

<213> Artificial Sequence

<220>
<223> epitope comprising SARS-CoV-2 sequence

<400> 100
Lys Lys Ile Ser Asn Cys Val Ala Asp Tyr Ser Val Leu Tyr Asn Ser
1               5                   10                  15
Thr

<210> 101
<211> 18
<212> PRT
<213> Artificial Sequence

<220>
<223> epitope comprising SARS-CoV-2 sequence

<400> 101
Lys Lys Ile Ser Asn Cys Val Ala Asp Tyr Ser Val Leu Tyr Asn Ser
1               5                   10                  15
Thr Phe

<210> 102
<211> 18
<212> PRT
<213> Artificial Sequence

<220>
<223> epitope comprising SARS-CoV-2 sequence

<400> 102
Lys Ser Phe Glu Ile Asp Lys Gly Ile Tyr Gln Thr Ser Asn Phe Arg
1               5                   10                  15
Val Val

<210> 103
<211> 17
<212> PRT
<213> Artificial Sequence

<220>
<223> epitope comprising SARS-CoV-2 sequence

<400> 103
Lys Ser Ile Val Ala Tyr Thr Met Ser Leu Gly Ala Asp Ser Ser Ile
1               5                   10                  15
Ala

<210> 104
<211> 17
<212> PRT
<213> Artificial Sequence

<220>
<223> epitope comprising SARS-CoV-2 sequence

<400> 104
Lys Thr Ser Val Asp Cys Asn Met Tyr Ile Cys Gly Asp Ser Thr Glu
1               5                   10                  15
Cys

<210> 105
<211> 17
<212> PRT
<213> Artificial Sequence

<220>
<223> epitope comprising SARS-CoV-2 sequence

<400> 105
Leu Ile Lys Asn Gln Cys Val Asn Phe Asn Phe Asn Gly Leu Thr Gly
1               5                   10                  15
Thr

<210> 106
<211> 17
<212> PRT
<213> Artificial Sequence

<220>
<223> epitope comprising SARS-CoV-2 sequence

<400> 106
Leu Lys Cys Ser Val Lys Ser Phe Glu Ile Asp Lys Gly Ile Tyr Gln
1               5                   10                  15
Thr

<210> 107
<211> 17
<212> PRT
<213> Artificial Sequence

<220>
<223> epitope comprising SARS-CoV-2 sequence

<400> 107
Leu Lys Gly Ala Cys Ser Cys Gly Ser Cys Cys Lys Phe Asp Glu Asp
1               5                   10                  15
Asp

<210> 108
<211> 17
<212> PRT
<213> Artificial Sequence

<220>
<223> epitope comprising SARS-CoV-2 sequence

<400> 108
Leu Leu Arg Ser Thr Ser Gln Lys Ser Ile Val Ala Tyr Thr Met Ser
1               5                   10                  15
Leu

<210> 109
<211> 17
<212> PRT
<213> Artificial Sequence

<220>
<223> epitope comprising SARS-CoV-2 sequence

<400> 109
Leu Gln Tyr Gly Ser Phe Cys Thr Gln Leu Asn Arg Ala Leu Ser Gly
1               5                   10                  15
Ile

<210> 110
<211> 17
<212> PRT
<213> Artificial Sequence

<220>
<223> epitope comprising SARS-CoV-2 sequence

<400> 110
Met Ala Tyr Arg Phe Asn Gly Ile Gly Val Thr Gln Asn Val Leu Tyr
1               5                   10                  15
Glu

<210> 111
<211> 17
<212> PRT
<213> Artificial Sequence

<220>
<223> epitope comprising SARS-CoV-2 sequence

<400> 111
Met Val Thr Ile Leu Leu Cys Cys Met Thr Ser Cys Cys Ser Cys Leu
1               5                   10                  15
Lys

<210> 112
<211> 17
<212> PRT
<213> Artificial Sequence

<220>
<223> epitope comprising SARS-CoV-2 sequence

<400> 112
Asn Ala Phe Asn Cys Thr Phe Glu Tyr Ile Ser Asp Ala Phe Ser Leu
1               5                   10                  15

Asp

<210> 113
<211> 17
<212> PRT
<213> Artificial Sequence

<220>
<223> epitope comprising SARS-CoV-2 sequence

<400> 113
Asn Val Phe Gln Thr Gln Ala Gly Cys Leu Ile Gly Ala Glu His Val
1               5                   10                  15
Asp

<210> 114
<211> 33
<212> PRT
<213> Artificial Sequence

<220>
<223> epitope comprising SARS-CoV-2 sequence

<400> 114
Pro Ala Ile Cys His Glu Gly Lys Ala Tyr Phe Pro Arg Glu Gly Val
1               5                   10                  15
Phe Val Phe Asn Gly Thr Ser Trp Phe Ile Thr Gln Arg Asn Phe Phe
            20                  25                  30
Ser

<210> 115
<211> 17
<212> PRT
<213> Artificial Sequence

<220>
<223> epitope comprising SARS-CoV-2 sequence

<400> 115
Pro Phe Ala Met Gln Met Ala Tyr Arg Phe Asn Gly Ile Gly Val Thr
1               5                   10                  15
Gln

<210> 116
<211> 19
<212> PRT
<213> Artificial Sequence

<220>
<223> epitope comprising SARS-CoV-2 sequence

<400> 116
Pro Val Ser Met Ala Lys Thr Ser Val Asp Cys Asn Met Tyr Ile Cys
1               5                   10                  15
Gly Asp Ser

<210> 117
<211> 17
<212> PRT
<213> Artificial Sequence

<220>
<223> epitope comprising SARS-CoV-2 sequence

<400> 117
Pro Trp Tyr Val Trp Leu Gly Phe Ile Ala Gly Leu Ile Ala Ile Val
1               5                   10                  15
Met

<210> 118
<211> 16
<212> PRT
<213> Artificial Sequence

<220>
<223> epitope comprising SARS-CoV-2 sequence

<400> 118
Arg Ala Ser Ala Asn Leu Ala Ala Thr Lys Met Ser Glu Cys Val Gly
1               5                   10                  15

<210> 119
<211> 17
<212> PRT
<213> Artificial Sequence

<220>
<223> epitope comprising SARS-CoV-2 sequence

<400> 119
Arg Asn Phe Thr Thr Ala Pro Ala Ile Cys His Glu Gly Lys Ala Tyr
1               5                   10                  15
Phe

<210> 120
<211> 17
<212> PRT
<213> Artificial Sequence

<220>
<223> epitope comprising SARS-CoV-2 sequence

<400> 120
Ser Gly Asn Cys Asp Val Val Ile Gly Ile Ile Asn Asn Thr Val Tyr
1               5                   10                  15
Asp

<210> 121

<211> 9
<212> PRT
<213> Artificial Sequence

<220>
<223> epitope comprising SARS-CoV-2 sequence

<400> 121
Ser Ile Val Ala Tyr Thr Met Ser Leu
1               5

<210> 122
<211> 15
<212> PRT
<213> Artificial Sequence

<220>
<223> epitope comprising SARS-CoV-2 sequence

<400> 122
Ser Thr Asp Leu Ile Lys Asn Gln Cys Val Asn Phe Asn Phe Asn
1               5                   10                  15

<210> 123
<211> 17
<212> PRT
<213> Artificial Sequence

<220>
<223> epitope comprising SARS-CoV-2 sequence

<400> 123
Ser Thr Phe Phe Ser Thr Phe Lys Cys Tyr Gly Val Ser Ala Thr Lys
1               5                   10                  15
Leu

<210> 124
<211> 17
<212> PRT
<213> Artificial Sequence

<220>
<223> epitope comprising SARS-CoV-2 sequence

<400> 124
Thr Ala Gly Trp Thr Phe Gly Ala Gly Ala Ala Leu Gln Ile Pro Phe
1               5                   10                  15
Ala

<210> 125
<211> 17
<212> PRT
<213> Artificial Sequence

<220>
<223> epitope comprising SARS-CoV-2 sequence

<400> 125
Thr Glu Cys Ala Asn Leu Leu Leu Gln Tyr Gly Ser Phe Cys Thr Gln
1               5                   10                  15
Leu

<210> 126
<211> 17
<212> PRT
<213> Artificial Sequence

<220>
<223> epitope comprising SARS-CoV-2 sequence

<400> 126
Val Ile Gly Ile Ile Asn Asn Thr Val Tyr Asp Pro Leu Gln Pro Glu
1               5                   10                  15
Leu

<210> 127
<211> 18
<212> PRT
<213> Artificial Sequence

<220>
<223> epitope comprising SARS-CoV-2 sequence

<400> 127
Val Tyr Tyr Pro Asp Glu Ile Phe Arg Ser Asp Thr Leu Tyr Leu Thr
1               5                   10                  15
Gln Asp

<210> 128
<211> 17
<212> PRT
<213> Artificial Sequence

<220>
<223> epitope comprising SARS-CoV-2 sequence

<400> 128
Tyr Ile Cys Gly Asp Ser Thr Glu Cys Ala Asn Leu Leu Leu Gln Tyr
1               5                   10                  15
Gly

<210> 129
<211> 17
<212> PRT
<213> Artificial Sequence

<220>
<223> epitope comprising SARS-CoV-2 sequence

<400> 129
Tyr Ser Val Leu Tyr Asn Ser Thr Phe Phe Ser Thr Phe Lys Cys Tyr

1               5                   10                  15
Gly

<210> 130
<211> 13
<212> PRT
<213> Artificial Sequence

<220>
<223> epitope comprising SARS-CoV-2 sequence

<400> 130
Cys Thr Phe Glu Tyr Ile Ser Asp Ala Phe Ser Leu Asp
1               5                   10

<210> 131
<211> 16
<212> PRT
<213> Artificial Sequence

<220>
<223> epitope comprising SARS-CoV-2 sequence

<400> 131
Gly Ala Ala Leu Gln Ile Pro Phe Ala Met Gln Met Ala Tyr Arg Phe
1               5                   10                  15

<210> 132
<211> 18
<212> PRT
<213> Artificial Sequence

<220>
<223> epitope comprising SARS-CoV-2 sequence

<400> 132
Lys Ser Ile Val Ala Tyr Thr Met Ser Leu Gly Ala Asp Ser Ser Ile
1               5                   10                  15
Ala Tyr

<210> 133
<211> 16
<212> PRT
<213> Artificial Sequence

<220>
<223> epitope comprising SARS-CoV-2 sequence

<400> 133
Met Ala Tyr Arg Phe Asn Gly Ile Gly Val Thr Gln Asn Val Leu Tyr
1               5                   10                  15

<210> 134

<211> 18
<212> PRT
<213> Artificial Sequence

<220>
<223> epitope comprising SARS-CoV-2 sequence

<400> 134
Asn Ala Phe Asn Cys Thr Phe Glu Tyr Ile Ser Asp Ala Phe Ser Leu
1               5                   10                  15
Asp Val

<210> 135
<211> 15
<212> PRT
<213> Artificial Sequence

<220>
<223> epitope comprising SARS-CoV-2 sequence

<400> 135
Ser Trp Phe Ile Thr Gln Arg Asn Phe Phe Ser Pro Gln Ile Ile
1               5                   10                  15

<210> 136
<211> 18
<212> PRT
<213> Artificial Sequence

<220>
<223> epitope comprising SARS-CoV-2 sequence

<400> 136
Ala Gly Cys Leu Ile Gly Ala Glu His Val Asp Thr Ser Tyr Glu Cys
1               5                   10                  15
Asp Ile

<210> 137
<211> 17
<212> PRT
<213> Artificial Sequence

<220>
<223> epitope comprising SARS-CoV-2 sequence

<400> 137
Leu Ile Ala Pro Arg Gly Tyr Phe Lys Ile Arg Ser Gly Lys Ser Ser
1               5                   10                  15
Ile

<210> 138
<211> 15
<212> PRT
<213> Artificial Sequence

<220>
<223> epitope comprising SARS-CoV-2 sequence

<400> 138
Gly Thr Ser Trp Phe Ile Thr Gln Arg Asn Phe Phe Ser Pro Gln
1               5                   10                  15

<210> 139
<211> 18
<212> PRT
<213> Artificial Sequence


<220>
<223> epitope comprising SARS-CoV-2 sequence

<400> 139
Met Met Cys Glu His Ile Tyr Tyr Thr Cys Val Arg Thr Ser Val Asp
1               5                   10                  15
Cys Cys


<210> 140
<211> 17
<212> PRT
<213> Artificial Sequence


<220>
<223> epitope comprising SARS-CoV-2 sequence

<400> 140
Tyr Thr Cys Val Arg Thr Ser Val Asp Cys Cys Met Lys Gly Ala Glu
1               5                   10                  15
Pro

## Description

### FIELD OF INVENTION

The invention relates to peptides derived from the spike glycoprotein of Severe Acute Respiratory Syndrome Coronavirus 2 (SARS-CoV-2 or 2019-nCoV) and nucleic acids encoding the same. These, and compositions comprising them can advantageously be used for vaccination against coronaviruses, in particular, as immunogenic antigens in vaccine formulations to fight SARS-CoV-2 and/or other coronaviruses. Such vaccines may advantageously also protect against further infectious agents, e.g., SARS-CoV and/or influenza.

### BACKGROUND ART

After causing an initial cluster of Pneumonia in Wuhan City, Hubei Province, SARS-CoV-2, the infective agent of CoVID-2019, has quickly spread through South East Asia and within a few weeks to Europe, Africa, and America. Initial estimates suggested a mortality rate of 2%. It is estimated that -18% of the cases show severe symptoms, although such estimates are still subject to rapid changes (https://www.who.int/news-room/detail/ 30-01-2020-statement-on-the-second-meeting-of-the-internationalhealth-regulations-(2005)-emergency-committee-regarding-theoutbreak-of-novel-coronavirus-(2019-ncov)). The recent emergence of the SARS-CoV-2 epidemic thus is a threat to the human population worldwide [1-3]. There are currently no proven therapies for this virus. By rule, production of an anti-viral vaccine implies lengthy testing procedures. Moreover, current approaches are mostly based on entire viral antigen(s) [4] and do not contemplate the risk deriving from cross-reactions due to the sharing of immune determinants between the virus and the human host [5]. Approaches that suggest peptide stretches from SARS-CoV-2 surface spike glycoprotein that are well conserved and surface exposed and are predicted to have reasonable epitope binding efficiency do not deal with the cross-reactivity potential of the suggested peptides [6].

As a matter of fact, cross-reactivity is a concrete risk [7], possibly leading to pathologic sequelae, in this way representing a main obstacle in using viral antigens for developing specific, effective, and safe anti-viral immunotherapeutics and diagnostic tools.

A further problem is represented by the tendency of viruses to mutate [8], so that a vaccine can be ineffective in a short time.

In light of these problems, the present invention addresses the need to provide advantageous compounds or compositions suitable for vaccination against coronaviruses such as SARS-CoV-2. This problem is solved by the subject-matter of the claims.

### SUMMARY OF THE INVENTION

The present invention provides a peptide comprising the sequence motif of any of SEQ ID NO: 1 (FAMQMAYRF), SEQ ID NO: 2 (FKCYGVS), or SEQ ID NO: 3 (IKWPWYI) and, preferably, combinations of such peptides.

The present invention also provides a peptide and combinations of peptides comprising the sequence motif of any of RGVYYPDK (SEQ ID NO: 4), NVTWFHA (SEQ ID NO: 5), FHAIH (SEQ ID NO: 6), PFNDG (SEQ ID NO:7), IRGWIF (SEQ ID NO: 8), IFGTT (SEQ ID NO: 9), VCEFQFC (10), CNDPF (SEQ ID NO: 11), VYYHK (SEQ ID NO: 12), NNKSW (SEQ ID NO:13), KSWM (SEQ ID NO: 14), WMESEF (SEQ ID NO: 15), YSSAN (SEQ ID NO: 16), CTFEY (SEQ ID NO: 17), GNFKN (SEQ ID NO:18), GYFKI (SEQ ID NO: 19), IYSKHT (SEQ ID NO: 20), PIGIN (SEQ ID NO: 21), GWTAG (SEQ ID NO: 22), AYYVG (SEQ ID NO: 23), NENGT (SEQ ID NO: 24), SETKC (SEQ ID NO: 25), GIYQT (SEQ ID NO: 26), VYAWNR (SEQ ID NO: 27), CVADY (SEQ ID NO: 28), STFKC (SEQ ID NO: 29), TNVYA (SEQ ID NO: 30), IADYN (SEQ ID NO: 31), DYNYKL (SEQ ID NO: 32), VIAWN (SEQ ID NO: 33), AW-NSNN (SEQ ID NO: 34), STPCN (SEQ ID NO: 35), PCNGV (SEQ ID NO: 36), GFNCYF (SEQ ID NO: 37), QSYGF (SEQ ID NO: 38), VKNKC (SEQ ID NO: 39), NKCVN (SEQ ID NO: 40), CVNFN (SEQ ID NO: 41), CTEVP (SEQ ID NO: 42), IGAEH (SEQ ID NO: 43), YQTQTN (SEQ ID NO: 44), IAYTMS (SEQ ID NO: 45), TSVDC (SEQ ID NO: 46), DCTMY (SEQ ID NO: 47), TMYICG (SEQ ID NO: 48), DSTEC (SEQ ID NO: 49), FCTQL (SEQ ID NO: 50), PIKDF (SEQ ID NO: 51), QYGDCL (SEQ ID NO: 52), GDCLG (SEQ ID NO: 53), DLICAQKF (SEQ ID NO: 54), MIAQY (SEQ ID NO: 55), SGWTF (SEQ ID NO: 56), WTFGA (SEQ ID NO: 57), FAMQM (SEQ ID NO: 58), MQMAYRF (SEQ ID NO: 59), RFNGI (SEQ ID NO: 60), MSECV (SEQ ID NO: 61), GYHLMS (SEQ ID NO: 62), KNFTT (SEQ ID NO: 63), PAICH (SEQ ID NO: 64), NGTHWFVTQ (SEQ ID NO: 65), TQRNF (SEQ ID NO: 66), NFYEP (SEQ ID NO: 67), IGIVN (SEQ ID NO: 68), NTVYD (SEQ ID NO: 69), YIWLGF (SEQ ID NO: 70), IAIVM (SEQ ID NO: 71), LCCMTS (SEQ ID NO: 72), MTSCC (SEQ ID NO: 73), CCKFD (SEQ ID NO: 74) or a combination of any of SEQ ID NO: 1-74).

These peptides are derived from the SARS-CoV-2 spike glycoprotein.

The inventors have found that said peptides (in particularly the peptides of any of SEQ ID NO: 1-3) have the following advantageous properties:
- they are absent in human proteins [9], so that immune responses against said peptides are highly likely to target only the SARS, e.g., SARS-CoV-2, Spike Glycoprotein without causing cross-reactions with human proteins.
- by being foreign to the human host, said peptides are potentially highly immunogenic and endowed with intrinsic adjuvant properties [10,11]
- finally, the peptide motifs of SEQ ID NO: 1-3 are highly conserved among coronaviruses (Table 3), so that, with any of these peptides, the present invention may allow for vaccination not only against SARS-CoV-2, but also against further, preferably, almost all coronavirus strains, in particular, those mentioned in Table 2. Of utmost importance, use of these highly conserved sequence motifs also allows for preparation of a vaccine that is likely to cover further mutations and variants of SARS. This also applies for SEQ ID NO: 58 and 59, which are subsequences of SEQ ID NO: 1).

The longer sequence motifs, e.g., SEQ ID NO: 1, 2 or 3 have the further advantage that it is more likely that antibodies can bind to the motif, and/or an epitope from the motif may be presented on MHC to elicit T cell immunity.

Indeed, as shown below, the inventors have shown that there are experimentally validated epitopes comprising said motifs, thus supporting the immunological potential of the motifs. These motifs were not previously connected with coronavirus infection, in particular, not with vaccination against a coronavirus (such as SARS-CoV-2), which is rendered possible by the present invention.

Additionally, all peptides of the present invention have several advantages over any other vaccine alternatives: peptides have short preparation times; low production costs; are easy to store and administer; a favorable safety profile, and they can be administered repeatedly. "Derived" in the context of being derived from SARS-CoV-2 spike glycoprotein points to the origin of the sequence, not necessarily the origin of the peptide. Thus, peptides of the invention may be easily synthesized. Alternatively, they may be produced by recombinant methods.

The peptides of the invention preferably comprise at most 30 consecutive amino acids occurring in the spike protein of a coronavirus and comprising the sequence of any of the recited sequence motifs, e.g., SEQ ID NO: 1-74 (or preferably, if the motifs are SEQ ID NO: 1-3, at most 30 consecutive amino acids occurring in the spike protein of a coronavirus and comprising the sequence of any of SEQ ID NO: 1-3).

For example, they may comprise at most 20 consecutive amino acids occurring in the spike protein of a coronavirus and comprising the sequence of any of the recited sequence motifs (e.g., SEQ ID NO: 1-74, or SEQ ID NO: 1-3). They may also comprise at most 10 consecutive amino acids occurring in the spike protein of a coronavirus and comprising the sequence of any of the recited sequence motif(s), e.g., any of SEQ ID NO: 1-74 (or SEQ ID NO: 1-3). For example, the peptides of the invention may comprise at most 7 (SEQ ID NO: 2 or 3), at most 8 (SEQ ID NO: 2 or 3), at most 9, at most 10, at most 11, at most 12, at most 13, at most 14, at most 15, at most 16, at most 17, at most 18, at most 19, at most 20, at most 21, at most 22, at most 23, at most 24, at most 25, at most 26, at most 27, at most 28, at most 29 or at most 30 consecutive amino acids occurring in the spike protein of a coronavirus and comprising the sequence of any of the recited sequence motifs (e.g., SEQ ID NO: 1-74).

Preferably, the peptides of the invention comprising the sequence motif of any of SEQ ID NO: 1-74 (preferably, SEQ ID NO: 1-3) comprise at most 30 consecutive amino acids occurring in the spike protein of a coronavirus, e.g., at most 20 consecutive amino acids occurring in the spike protein of a coronavirus, optionally, at most 10 consecutive amino acids occurring in the spike protein of a coronavirus. For example, the peptides of the invention may comprise at most 7 (e.g., SEQ ID NO: 2 or 3), at most 8 (e.g., SEQ ID NO: 2 or 3), at most 9 (e.g., SEQ ID NO: 1-3), at most 10, at most 11, at most 12, at most 13, at most 14, at most 15, at most 16, at most 17, at most 18, at most 19, at most 20, at most 21, at most 22, at most 23, at most 24, at most 25, at most 26, at most 27, at most 28, at most 29 or at most 30 consecutive amino acids occurring in the spike protein of a coronavirus, e.g., exactly that number.

In another embodiment, the peptide of the invention may comprise other parts from the spike protein of a coronavirus, as long as the consecutive passage comprising any of the recited sequence motifs, e.g., SEQ ID NO: 1-74 (or, preferably, SEQ ID NO: 1-3) is not longer than the recited number of consecutive amino acids, e.g., at most 30, at most 20 or at most 10 consecutive amino acids.

In one embodiment, the peptide does not comprise any complete naturally occurring coronavirus-derived protein. Alternatively, the peptide of the invention may be linked to a coronavirus protein other than the spike protein or sequences from such another protein. Such sequences may be of any length, e.g., up to 30 amino acids or longer than 30 amino acids. They may e.g., be derived from the nucleocapsid protein or a non-structural polyprotein of SARS-CoV and/or the corresponding peptides or proteins of SARS-CoV-2. Peptides from the non-structural polyprotein 1a of SARS-CoV and nucleocapsid-derived peptides have been previously shown to be able of producing a CTL-response [17], and such peptides may, e.g., be used in this context.

However, it is preferred that the peptide of the invention consists of the sequence motif of any of SEQ ID NO: 1-74, or a combination thereof, preferably, of the sequence motif of any of SEQ ID NO: 1-3, e.g., of the peptide consisting of SEQ ID NO: 1, the peptide consisting of SEQ ID NO: 2, or the peptide consisting of SEQ ID NO: 3, or a combination thereof. As all recited sequences of SE ID NO: 1-74 do not occur in the human proteome, this significantly reduces the risk of cross-reactivities.

The peptide of the invention may comprise a combination of peptides consisting of any of SEQ ID NO: 1-3 (or consist of said combination), e.g., a fusion protein consisting exclusively of the sequences of any of SEQ ID NO: 1, 2 or 3.

The peptide of the invention may comprising a plurality of peptides of the invention. For example, the peptide of the invention may comprise a plurality of repeats of any of SEQ ID NO: 1-74, e.g., a plurality of repeats of SEQ ID NO: 1, a plurality of repeats of SEQ ID NO: 2 and/or plurality of repeats of SEQ ID NO: 3, or it may consist of said plurality of repeats.

The repeats may, e.g., be spaced in a distance allowing for recognition of B cell receptors. Cross-linking of B cell receptors is known to activate B cells to produce antibodies even in the absence of T cell help. To this end, the length of the sequence of consecutive amino acids comprising SEQ ID NO: 1-74, preferably, SEQ ID NO: 1, 2 or 3 may be chosen appropriately, or spacer amino acids may be included in the peptide of the invention. The spacer preferably consists of peptide sequences non present in the human proteome [22]. The peptide may comprising a plurality of repeats of the same sequence motif of the invention (i.e., SEQ ID NO: 1, 2 or 3), which is advantageous for cross-linking B cell receptors.

Alternatively, or additionally, the peptide of the invention may comprise at least two different sequence motifs of the invention, e.g., two peptides of the invention comprising different sequence motifs. A preferred peptide of the invention may comprise at least 2 peptides comprising SEQ ID NO: 1, 2 or 3, e.g., SEQ ID NO: 1 and SEQ ID NO: 2, SEQ ID NO: 2 and SEQ ID NO: 3 or SEQ ID NO: 1 and SEQ ID NO: 3. The peptide may optionally comprise peptides of the invention comprising SEQ ID NO: 1, 2 and 3.

In one embodiment, the peptide of the invention comprises SEQ ID NO: 1. In one embodiment, the peptide of the invention comprises SEQ ID NO: 2. In one embodiment, the peptide of the invention comprises SEQ ID NO: 3.

To generate long-lasting immunity, it is helpful if the peptide comprises at least one T-cell epitope. B-cell epitopes may induce antibodies, e.g., neutralizing antibodies. Preferred peptides of the invention are experimentally validated epitopes. Sequences of such peptides (SEQ ID NO: 75-140) are shown in Table 1 below. Peptides comprising SEQ ID NO: 89, 115 or 131 comprise the highly conserved SEQ ID NO: 1 and are therefore particularly preferred.

**Table 1: Immunologic potential of peptides of the invention (consecutive amino acid sequences present in SARS CoV-2 and absent from *Homo sapiens* at the pentamer level are capitalized).** Data from: IEDB (Immune epitope database) [18]. Epitopic sequences containing at least one pentapeptide from the SEQ ID NO: 1-74 and validated in B- and/or T-cells assays are reported. Underlined font indicates positivity in T-cell assays. **Bold** indicates positivity in both in B and T-cell assays. Normal font indicates positivity in B-cell assays.

Peptides of the invention preferably comprise at least one epitope capable of binding to a human MHC molecule, e.g., MHC class II molecule, and eliciting a T cell response, e.g., a helper T cell response. A cytotoxic response may also be helpful. Said epitope may be derived from a SARS protein, or from a different immunogenic protein, e.g., as described below. It is also advantageous if a peptide comprises both B and T cell epitopes.

The peptide of the invention may be conjugated to a carrier protein, preferably, an immunogenic protein derived from an infectious agent. In one embodiment, the peptide of the invention may be a fusion peptide (or a fusion protein, as "peptide" and "protein" are used synonymously herein) comprising an immunogenic protein.

| SEQ ID NO: | IEDB-ID-Number - Epitopic sequences containing the unique SARS CoV-2 Spike Glycoproteins pentapeptides | |
|---|---|---|
| 75 | 307 | aalvsgtatagWTFGAg |
| 76 | 462 | aatkMSECVlgqskrvd |
| 77 | 1460 | agcllGAEHvdtsyecd |
| 78 | 3176 | aMQMAYRF |
| 79 | 6011 | canlllqygsFCTQLnralsgia |
| 80 | 6333 | cgpklstdliknqCVNFNfngltgtgvltpsskrfqpfqqfg |
| 81 | 6334 | cgpklstdliknqCVNFNfngltgtgvltpsskrfqpfqqfgrdvsdftd |
| 82 | 7066 | csqnplaelkcsvksfeidkGIYQTsnfrvvpsgd |
| 83 | 7217 | cttfddvqapnytqhtssmRGVYYPDeifr |
| 84 | 7383 | CYGVSatklndlcfsnv |
| 85 | 8239 | dfcgkGYHLMSfpqaap |
| 86 | 12417 | eidkGIYQTsnfrvvps |
| 87 | 15903 | ffSTFKCYGVSatklnd |
| 88 | 18161 | fvfngtswfiTQRNFfs |
| 89 | 18515 | gaalqipFAMQMAYRFn |
| 90 | 21464 | gnliaprGYFKlrsqkssim |
| 91 | 22321 | gsFCTQLn |
| 92 | 24978 | htssmRGVYYPDeifrs |
| 93 | 25250 | IADYNYKLpddfmgcvl |
| 94 | 25293 | iagllAIVMvtillccm |
| 95 | 25378 | iapgqtgvlADYNYKLp |
| 96 | 25382 | iaprGYFKlrngkssimrsdapigtcssecit |
| 97 | 29728 | iywtivkpqdillinstgnliaprGYFKlrn |
| 98 | 30987 | kGIYQTsn |
| 99 | 30988 | kGIYQTsnfrvvpsgdvvrf |
| 100 | 31581 | kkisnCVADYsvlynst |
| 101 | 31582 | kkisnCVADYsvlynstf |
| 102 | 33305 | ksfeidkGIYQTsnfrvv |
| 103 | 33358 | ksivAYTMSlgadssia |
| 104 | 33874 | kTSVDCnMYICGDSTEC |
| 105 | 36579 | liknqCVNFNfngltgt |
| 106 | 36815 | lkcsvksfeidkGIYQT |
| 107 | 36856 | lkgacscgsCCKFDedd |
| 108 | 37758 | **llrstsqksivAYTMSI** |
| 109 | 39023 | lqygsFCTQLnralsgi |
| 110 | 41177 | MAYRFNGlgvtqnvlye |
| 111 | 42999 | mvtilLCCMTSCCsclk |
| 112 | 43145 | nafnCTFEYisdafsld |
| 113 | 46379 | nvfqtqagcllGAEHvd |
| 114 | 46822 | PAICHegkayfpregvfvfngtswfiTQRNFfs |
| 115 | 47479 | pFAMQMAYRFNGlgvtq |
| 116 | 49968 | pvsmakTSVDCnMYICGds |
| 117 | 50058 | pwyvwlgfiagllAIVM |
| 118 | 53202 | rasanlaatkMSECVg |
| 119 | 54989 | rnfttaPAICHegkayf |
| 120 | 58143 | sgncdvvigiinNTVYD |
| 121 | 58730 | sivAYTMSI |
| 122 | 61554 | stdliknqCVNFNfn |
| 123 | 61598 | stffSTFKCYGVSatkl |
| 124 | 62872 | tagWTFGAgaalqipfa |
| 125 | **63309** | **tecanlllqygsFCTQL** |
| 126 | 68971 | vigiinNTVYDplqpel |
| 127 | 72205 | VYYPDeifrsdtlyltqd |
| 128 | 74173 | yicgDSTECanlllqyg |
| 129 | 75920 | ysvlynstffSTFKCYG |
| 130 | 99918 | CTFEYisdafsld |
| 131 | 100048 | gaalgipFAMQMAYRF |
| 132 | 100230 | ksivAYTMSlqadssiay |
| 133 | 100300 | MAYRFNGlgvtgnvly |
| 134 | 100316 | nafnCTFEYisdafsldv |
| 135 | 100537 | swfiTQRNFfspqii |
| 136 | 100711 | agcllGAEHvdtsyecdi |
| 137 | 129239 | liaprGYFKlrsgkssi |
| 138 | 532052 | gtswfiTQRNFfspg |
| 139 | 873061 | mmcehiyytcvrTSVDCc |
| 140 | 874104 | ytcvrTSVDCcmkgaep |

The immunogenic protein is preferably derived from an infectious agent and selected from the group comprising influenza hemagglutinin, diphteria toxoid, pertussis toxoid, tetanus toxoid and hepatitis B virus surface antigen. This conjugation or fusion may not only contribute to immunogenicity, in particular, towards induction of an immune response targeting a sequence motif of the invention, and thus allowing for vaccination against a coronavirus, but it may further allow for simultaneous induction of immune responses against the infectious agent from which the immunogenic protein is derived. For example, vaccination with a conjugate or fusion protein of influenza hemagglutinin and a coronavirus peptide of the invention may also allow for vaccination against at least one coronavirus, e.g., SARS-CoV-2 and/or SARS-CoV and influenza.

The peptide of the invention may be administered in combination with peptides derived from other pathogens (influenza virus, measles virus, toxoplasma et alia), preferably, peptides that are present only in the pathogens and are absent in the human host.

Toxoids (or toxins) may be inactivated either by chemical treatment or genetic modification, as known in the art. The conjugate or fusion peptide may further be part of a conjugate vaccine, wherein the fusion peptide is also conjugated to a polysaccharide moiety, e.g., for vaccination against *Neisseria meningitidis, Streptococcus pneumoniae* or *Haemophilus influenzae.*

The present invention also provides nucleic acids encoding the peptide of the invention, which are preferably based on optimal codon usage. The nucleic acid may be RNA or DNA, preferably, DNA. The nucleic acid may be an expression vector suitable for producing the peptide(s) of the invention. This production may be, e.g., in cell culture (e.g., in human cells) or in eggs. Codon usage is selected in an appropriate manner for the site of production, e.g., codon usage is optimized for production in humans if the nucleic acid is to be expressed in human cells.

The nucleic acid may also be suitable for nucleic acid vaccination, e.g., for injection into a human subject and production of the peptide of the invention in said subject. The nucleic acid may also be a virus, e.g., suitable for vaccination of a human subject. In a virus or an expression vector, the nucleic acid sequence encoding the peptide of the invention is functionally linked to a heterologous promotor, e.g., a promotor suitable for expression in human cells, e.g., in human skin and/or human mucosa. A heterologous promotor is not a coronavirus promotor.

If the nucleic acid is intended for nucleic acid vaccination, it may e.g., comprise CpG oligonucleotides, which have been shown to exert an adjuvant effect.

The invention further provides a composition comprising the peptide of the invention. In one embodiment, such a composition comprises peptides consisting of any of SEQ ID NO: 1-74, preferably, of any of SEQ ID NO: 1-3. A preferred composition of the invention comprises peptides comprising SEQ ID NO: 1, 2 and 3, e.g., peptides consisting of SEQ ID NO: 1, 2 and 3.

The invention further provides a composition comprising a nucleic acid of the invention, e.g., a combination of nucleic acids of the composition comprising two or more of the nucleic acids of the invention encoding different sequence motifs any of SEQ ID NO: 1-74, preferably, at least one, more preferably, two, most preferably, three of SEQ ID NO: 1-3.

It has been shown that short (e.g., pentameric) non-human peptide sequences not found in the universal (human) genome can enhance antigen specific responses and serve as adjuvants in vaccines [11, 19, 20]. This was e.g., shown by Patel et al. in [11] for the example of a fusion protein of an influenza hemagglutinin expressed as a C-terminal fusion protein with the pentamer sequences disclosed therein or for combinations of the influenza hemagglutinin with the pentamer provided separately. However, Patel et al. only showed immunity against influenza. They did not envision that such vaccines can also be used for vaccination against the source of the unique sequences. In contrast, the present invention provides a vaccine against both the coronavirus from which the unique sequence motifs of the invention are derived (e.g., against SARS-CoV-2) and against other antigens that may be provided as conjugates, fusion proteins or combinations, e.g., influenza antigens. For example, any of the vaccine constructs of Patel or a corresponding construct using another influenza hemagglutinin of interest may be used, wherein the pentamer used is exchanged against a sequence motif of the present invention, namely any of SEQ ID NO: 1-74. Preferably, a sequence motif of SEQ ID NO: 1-3 is used, which, due to the conserved nature, allows for vaccination against a plurality of coronaviruses, as disclosed herein, in addition to vaccination against influenza.

In one embodiment, and in accordance with the results of Patel et al. [11] or [19, 20], the composition of the present invention does not comprise an adjuvant (except for the sequence motif of any of SEQ ID NO: 1-74).

It has however also been shown by Patel et al. [11] that the short immunomodulatory peptides can act synergistically with other adjuvants. This also applies to the peptides of the present invention. Thus, the invention also provides a composition comprising the peptide of the invention or the nucleic acid of the invention, wherein the composition further comprises at least one adjuvant. Exemplary adjuvants are, e.g., provided in [21]. The adjuvant may be, e.g., aluminum hydroxide, aluminum phosphate, alum (aluminum sulfate), MF59 (an o/w emulsion with squalene oil dispersed with the help of surfactants polysorbate 80 and sorbitan oleate), monophosphoryl lipid A (MPL), Adjuvant system 04 (AS04, i.e., monophosphoryl lipid A adsorbed on aluminum hydroxide), CpG oligonucleotides, polylCLC, QS21 (a purified saponin extract), ISCOMs, e.g., ISCOMATRIX adjuvant, Freund's complete adjuvant (a w/o emulsion of a mineral oil, paraffin and killed mycobacteria), Freund's incomplete adjuvant (a w/o emulsion of a mineral oil, paraffin without mycobacteria), Montanide ISA720, Montanide ISA51 (both w/o emulsions with squalene as the oil and mono-oleate as the surfactant), Adjuvant system 02 (AS02, i.e., comprising squalene and two hydrophobic immune adjuvants, MPL and QS-21), liposomes or another short immunomodulatory peptide (e.g., as disclosed by [19, 20]), or a nucleic acid encoding the same. Preferably, the adjuvant is admitted for use in human subjects.

The composition may e.g., be an aqueous solution, e.g., a water-based solution. The solution may also comprise a physiological buffer, e.g., PBS, or a saline such as Ringer solution. The composition preferably is suitable for administration to a human subject. The solution may contain a preservative, or may be devoid of preservatives.

Alternatively, the solution may be dry, e.g., in lyophilized or spray-dried form. Before use, it can be reconstituted, e.g., in water for injection. A dry form of the composition of the invention may, e.g., comprise a bulking agent such as a sugar, e.g., trehalose.

In a preferred embodiment, the invention provides a solution comprising peptides consisting of any of SEQ ID NO: 1-3, preferably, all three of these peptides, e.g., in equal amounts. One dose may comprise about 10-500 µg, e.g., 100 µg of each peptide, for example, in 0.2 mL aqueous solution, e.g., PBS. However, peptide concentrations can be easily adapted by the skilled person.

The invention also provides a pharmaceutical composition comprising the peptide of the invention, the nucleic acid of the invention or the composition of the invention. Preferably, said pharmaceutical composition is a vaccine. The pharmaceutical composition is for use in vaccination against at least one coronavirus, preferably, against SARS-CoV-2. As disclosed herein, preferred pharmaceutical compositions, in particular, comprising peptides having a sequence motif of any of SEQ ID NO: 1-3 or nucleic acids encoding the same, may advantageously be used in vaccination against at least SARS-CoV-2 and SARS-CoV, preferably, for use in vaccination against all coronaviruses mentioned in Table 2 below. Moreover, SEQ ID NO 3 is partially (6 consecutive residues out of 7) shared between SARS-CoV-2 and MERS-CoV, therefore a vaccine comprising SEQ ID NO 3 may also be effective against MERS-CoV.

The vaccine may be for prevention of coronavirus infection, i.e., for reducing the incidence or coronavirus infection, e.g., SARS-CoV-2. The vaccine may also be useful for therapeutic vaccination of a subject infected with a coronavirus, or suspected of being infected with a coronavirus, e.g., SARS-CoV-2.

The pharmaceutical composition of the invention may further comprise or encode peptides from an influenza antigen or influenza antigens, e.g., influenza hemagglutinin. Thus, it may be for use in vaccination against at least one coronavirus such as SARS-CoV-2 and influenza.

The subject to be immunized typically is a mammal, e.g., a human subject. It may be a subject at particular risk from the coronavirus infection, e.g., a subject 60 or more years old, and/or the subject may be a male subject, a subject having diabetes, a subject having a heart disease, a subject having a lung disease. Male subjects of 60 or more years having co-morbidities, e.g., as stated are at particular risk, and thus preferably vaccinated. Elderly patients are also a particular risk group for influenza, so it is particularly advantageous to provide a combination vaccine.

Administration of the vaccine of the invention may be, e.g., intranasal, transdermal, intravenous, subcutaneous or intramuscular. Peptide or nucleic acid concentration and times and number of booster vaccinations can easily be modulated and standardized depending on the subject age, weight and first response. Preferably, for preventive vaccination, the vaccine is repeatedly administered, but a single administration is also possible.

The invention further provides a method of vaccination against at least one coronavirus (e.g., SARS-CoV-2), comprising administering the vaccine of the invention to a subject. The invention also provides a method of treating a coronavirus infection (e.g., with SARS-CoV-2), or treating a subject suspected of having a coronavirus infection (e.g., with SARS-CoV-2), comprising administering the vaccine of the invention to the subject. In addition, a method of preparing a vaccine against a coronavirus infection is provided, comprising formulating a peptide or nucleic acid as disclosed herein as a vaccine suitable for human administration. Said method may further comprise synthesizing said peptide or nucleic acid.

Further, the invention discloses a method of preparing a peptide of the invention, in particular, of preparing a vaccine of the invention, comprising synthesizing a peptide or nucleic acid of the invention, and, optionally packaging the peptide or nucleic acid of the invention. The invention also provides a syringe comprising a peptide or nucleic acid or composition of the invention, which may be for use in vaccinating a subject, preferably, a human subject, against a coronavirus such as SARS-CoV-2.

In conclusion, the invention teaches the use of unique coronavirus peptides comprising any of SEQ ID NO: 1-74, or a combination thereof, in particular, the three peptides of SEQ ID NO: 1-3, derived from the spike glycoprotein of Severe Acute Respiratory Syndrome Coronavirus 2 (SARS-CoV-2) to be used as immunogenic antigens in vaccine formulations to fight SARS-CoV-2 and coronaviruses in general.

### REFERENCES

1. Zhu, N. et al. A novel coronavirus from patients with pneumonia in China. N. Engl.J. Med. https://doi.org/10.1056/NEJMoa2001017 (2019)
2. Gralinski, L. E. & Menachery, V. D. Return of the Coronavirus: 2019-nCoV. Viruses 12.2, 135 (2020).
3. Huang, C. et al. Clinical features of patients infected with 2019 novel coronavirus in Wuhan, China. Lancet 395, 497-506 (2020).
4. Enjuanes L, et al.. Molecular Basis of Coronavirus Virulence and Vaccine Development. Adv Virus Res. 2016;96:245-286.
5: Kanduc D. Peptide cross-reactivity: the original sin of vaccines. Front Biosci 2012;4: 1393-1401.
6. Tahir ul Qamar, M., et al. Epitope-based peptide vaccine design and target site depiction against Middle East Respiratory Syndrome Coronavirus: an immune-informatics study. J Transl Med 17, 362 (2019).
7: Kanduc D, Stufano A, Lucchese G, Kusalik A. Massive peptide sharing between viral and human proteomes. Peptides. 2008 Oct;29(10): 1755-66.
8: Drake JW. Rates of spontaneous mutation among RNA viruses. Proc Natl Acad Sci U S A. 1993 May 1;90(9):4171-5.
9. Lucchese G. Epitopes for a 2019-nCoV vaccine. Cell Mol Immunol. doi.org/10.1038/s41423-020-0377-z
10. Kanduc D. "Self-nonself" peptides in the design of vaccines. Curr Pharm Des. 2009; 15(28):3283-9.
11. Patel, A. et al. Pentamers not found in the universal proteome can enhance antigen specific immune responses and adjuvant vaccines. PLoS ONE 7, e43802, https://doi.org/10.1371/journal.pone.0043802 (2012).
12. Chen C, et al., UniProt Consortium, A fast Peptide Match service for UniProt Knowledgebase, Bioinformatics, 2013, 29, 2808-2809.
13. Almofti, Y. A., et al. (2018). Multi Epitopes Vaccine Prediction against Severe Acute Respiratory Syndrome (SARS) Coronavirus Using Immunoinformatics Approaches. American Journal of Microbiological Research, 6(3), 94-114.
14. Ahmed, S.F.; et al.. Preliminary Identification of Potential Vaccine Targets for the COVID-19 Coronavirus (SARS-CoV-2) Based on SARS-CoV Immunological Studies. Viruses 2020, 12, 254.
15. Bojin, F. et al. (2020). Design of an Epitope-Based Synthetic Long Peptide Vaccine to Counteract the Novel China Coronavirus (2019-nCoV). https://www.preprints.org/manuscript/202002.0102/v1.
16. Miysaa I. et al. Design of multi epitope-based peptide vaccine against E protein of human 2019-nCoV: An immunoinformatics approach. bioRxiv 2020.02.04.934232; doi: https://doi.org/10.1101/2020.02.04.934232.
17. Kohyama, S. et al., (2009). Efficient induction of cytotoxic T lymphocytes specific for severe acute respiratory syndrome (SARS)-associated coronavirus by immunization with surface-linked liposomas peptides derived from a non-structural polyprotein a. Antivir. Res. 84:168-177.
18. Vita, R. et al., (2010). The immune epitope database 2.0. Nucleic Acids Res. 38, D854-D862.
19. Dong J.C. et al., (2012). Hypothesis driven development of new adjuvants. Human vaccines& Immunotherapeutics 9(4):808-811.
20. Nagpal, G. (2018). Computer-aided prediction of antigen presenting cell modulators for designing peptide-based adjuvants. J Tranl Med 16:181.
21. Li, 1. et al. (2014). Peptide Vaccine: Progress and challenges. Vaccines 2:515-536.
22. Kanduc D. Oligopeptides for Immunotherapy Approaches in Ovarian Cancer Treatment. Curr Drug Discov Technol. 2019;16(3):285-289.

### Examples

To facilitate the swift development of a candidate vaccine for SARS-CoV-2 (also designated 2019-nCoV), the inventors compared the viral and the human proteomes, searching for short peptides, at least pentapeptides, that are unique to the pathogen.

They followed the rationale that non-self sequences are highly immunogenic and uniquely viral epitopes should improve safety and efficacy by minimizing the risk for cross-reactions and increasing anti-viral specificity. The analysis was conducted on the entire viral proteome, but primarily focused on the surface spike glycoprotein (id = "QHD43416.1), because the immune response against it is highly likely to exert a neutralizing effect. The entire amino acid (aa) sequence of the 2019-nCoV was retrieved from https://www.ncbi.nlm.nih.gov/nuccore/MN908947 and dissected into short overlapping peptides. Then, each peptide was analyzed for occurrences in the human proteome using the Peptide Match program (https://research.bioinformatics.udel.edu/peptidematch/ index.jsp) [12].

The inventors showed that 933 viral peptides (at least pentapeptides) are absent from the human proteome, and therefore foreign to the human immune system. Among these non-self peptides, 107 are embedded in the viral surface glycoprotein (spike protein) that mediates binding to the human ACE2 and cellular entry. The recommended oligopeptides for a multi-epitope 2109- nCoV-vaccine are peptides of SEQ ID NO: 1-74 as disclosed above, and they are also partly shown in Table 1, Panel a of [9].

Three points need to be stressed. First, short peptides that are foreign to the host immune system have been experimentally validated not only as positive immunomodulants (i.e., adjuvants) in conjunction to vaccines, but are also evidenced as providing direct protection against lethal viral infections, at least in animal models [11].

Second, searching for the 107 human-foreign spike protein pentapeptides in the Immune Epitope Database (IEDB; www.iedb.org) yielded a list of n = 66 epitopes (Table 1, above). The IEDB is a publicly available, curated epitope repository. The presence of a peptide sequence in the IEDB indicates that it has a recognized and experimentally proven immunologic relevance. These results provide experimental proof for the immunogenic potential of the non-self peptides identified in the present study through comparative *Homo sapiens-*coronavirus proteome analysis.

Third, an immune response induced by the spike protein oligopeptides that are absent in the human proteins would exert a neutralizing effect on the coronavirus, in light of the evidence for the surface glycoprotein as a ligand for the human ACE2 in viral entry processes.

Furthermore, the inventors identified particularly advantageous peptides, namely, peptides comprising a sequence motif of any of SEQ ID NO: 1-3, wherein said sequence motifs are highly conserved among different coronaviruses. They are thus also suitable for vaccination against a plurality of different coronaviruses, and it is likely that said peptides are not easily mutated, so that vaccination against different serotypes and future mutated types of coronaviruses such as SARS is also possible.

Previously published research from the inventors and other groups has already proposed peptide sequences for vaccines against the SARS [13] and SARS-CoV-2 [9,14-16] viruses. The solution here described differs substantially in the following aspects:
1. The peptides of the present invention have been specifically selected in light of their absence from the human proteome, that is to say that they are foreign to the human immune system or "non-self". This hinders cross-reaction and molecular mimicry and makes the vaccine here proposed safe.
2. The peptides comprising any of SEQ ID NO. 1-3 and combinations thereof here presented have been selected on the basis of their high degree of conservation among coronaviruses. This should also make the proposed vaccine effective against different serotypes and against possible mutations of the viruses.
3. The "non-self" status makes the peptides here presented highly immunogenic and at the same time confers adjuvant properties. The vaccine here described does not need any no additional adjuvant. A coronavirus vaccine free of additional adjuvants has not been mentioned or proposed in any previous publication, and no currently available vaccine is composed exclusively of peptides without additional adjuvants.

**Table 2. Conservation of the Spike Glycoprotein Peptides of the Invention (namely, FAMQMAYRF (SEQ ID NO: 1), FKCYGVS (SEQ ID NO: 2), and IKWPWYI (SEQ ID NO: 3) in Coronaviruses. Data obtained using Pir Peptide Match program at research.bioinformatics.udel.edu/ [12] NCBI Taxld in parentheses.**

| **Peptide** | **Coronavirus type/strain** |
|---|---|
| FAMQMAYRF | Rhinolophus affinis coronavirus [1487703] |
| FAMQMAYRF | Rhinolophus affinis coronavirus [1487703] |
| FAMQMAYRF | Bat SARS-like coronavirus [1508227] |
| FAMQMAYRF | Bat SARS-like coronavirus YNLF_34C [1699361] |
| FAMQMAYRF | Bat SARS-like coronavirus YNLF_31C [1699360] |
| FAMQMAYRF | SARS-like coronavirus BatCoV/BB9904/BGR/2008 [1737344] |
| FAMQMAYRF | SARS-like coronavirus WIV16 [1739625] |
| FAMQMAYRF | Bat coronavirus [1508220] |
| FAMQMAYRF | Bat coronavirus [1508220] |
| FAMQMAYRF | Bat coronavirus [1508220] |
| FAMQMAYRF | Bat coronavirus [1508220] |
| FAMQMAYRF | Human SARS coronavirus (SARS-CoV) [694009] |
| FAMQMAYRF | Bat coronavirus [1508220] |
| FAMQMAYRF | Bat SARS-like coronavirus [1508227] |
| FAMQMAYRF | Bat SARS-like coronavirus [1508227] |
| FAMQMAYRF | Bat SARS-like coronavirus [1508227] |
| FAMQMAYRF | Bat SARS-like coronavirus [1508227] |
| FAMQMAYRF | Bat SARS-like coronavirus [1508227] |
| FAMQMAYRF | Bat SARS-like coronavirus [1508227] |
| FAMQMAYRF | Bat SARS-like coronavirus [1508227] |
| FAMQMAYRF | Bat SARS-like coronavirus [1508227] |
| FAMQMAYRF | Bat SARS-like coronavirus [1508227] |
| FAMQMAYRF | Bat SARS-like coronavirus [1508227] |
| FAMQMAYRF | Bat SARS-like coronavirus [1508227] |
| FAMQMAYRF | Bat SARS-like coronavirus [1508227] |
| FAMQMAYRF | Bat SARS-like coronavirus [1508227] |
| FAMQMAYRF | SARS coronavirus Urbani [228330] |
| FAMQMAYRF | SARS coronavirus Urbani [228330] |
| FAMQMAYRF | Human SARS coronavirus (SARS-CoV) [694009] |
| FAMQMAYRF | Coronavirus BtRI-BetaCoV/SC2018 [2591233] |
| FAMQMAYRF | Coronavirus BtRs-BetaCoV/YN2018B [2591235] |
| FAMQMAYRF | Coronavirus BtRs-BetaCoV/YN2018D [2591237] |
| FAMQMAYRF | Coronavirus BtRs-BetaCoV/YN2018A [2591234] |
| FAMQMAYRF | Coronavirus BtRs-BetaCoV/YN2018C [2591236] |
| FAMQMAYRF | SARS coronavirus Frankfurt1-v01 [391355] |
| FAMQMAYRF | SARS coronavirus WF188 [385687] |
| FAMQMAYRF | SARS coronavirus ES191 [385685] |
| FAMQMAYRF | SARS coronavirus ES260 [385686] |
| FAMQMAYRF | SARS coronavirus CS21 [385683] |
| FAMQMAYRF | SARS coronavirus CS24 [385684] |
| FAMQMAYRF | Human SARS coronavirus (SARS-CoV) [694009] |
| FAMQMAYRF | SARS coronavirus BJ182a [511432] |
| FAMQMAYRF | SARS coronavirus BJ182b [511433] |
| FAMQMAYRF | SARS coronavirus BJ182-4 [511430] |
| FAMQMAYRF | SARS coronavirus BJ182-8 [511431] |
| FAMQMAYRF | SARS coronavirus BJ182-12 [511429] |
| FAMQMAYRF | SARS coronavirus P2 [627442] |
| FAMQMAYRF | SARS coronavirus Rs_672/2006 [722424] |
| FAMQMAYRF | SARS coronavirus ExoN1 [627440] |
| FAMQMAYRF | SARS coronavirus wtic-MB [698419] |
| FAMQMAYRF | SARS coronavirus ExoN1 [627440] |
| FAMQMAYRF | SARS coronavirus ExoN1 [627440] |
| FAMQMAYRF | SARS coronavirus ExoN1 [627440] |
| FAMQMAYRF | SARS coronavirus ExoN1 [627440] |
| FAMQMAYRF | SARS coronavirus MA15 ExoN1 [633140] |
| FAMQMAYRF | SARS coronavirus MA15 ExoN1 [633140] |
| FAMQMAYRF | SARS coronavirus ExoN1 [627440] |
| FAMQMAYRF | SARS coronavirus MA15 [633137] |
| FAMQMAYRF | SARS coronavirus wtic-MB [698419] |
| FAMQMAYRF | SARS coronavirus ExoN1 [627440] |
| FAMQMAYRF | SARS coronavirus MA15 ExoN1 [633140] |
| FAMQMAYRF | SARS coronavirus ExoN1 [627440] |
| FAMQMAYRF | SARS coronavirus ExoN1 [627440] |
| FAMQMAYRF | SARS coronavirus HKU-39849 [228404] |
| FAMQMAYRF | SARS coronavirus HKU-39849 [228404] |
| FAMQMAYRF | Bat SARS coronavirus HKU3-4 [742001] |
| FAMQMAYRF | Bat SARS coronavirus HKU3-5 [742002] |
| FAMQMAYRF | Bat SARS coronavirus HKU3-6 [742003] |
| FAMQMAYRF | Bat SARS coronavirus HKU3-7 [742004] |
| FAMQMAYRF | Bat SARS coronavirus HKU3-8 [742005] |
| FAMQMAYRF | Bat SARS coronavirus HKU3-9 [742006] |
| FAMQMAYRF | Bat SARS coronavirus HKU3-10 [741997] |
| FAMQMAYRF | Bat SARS coronavirus HKU3-11 [741998] |
| FAMQMAYRF | Bat SARS coronavirus HKU3-12 [741999] |
| FAMQMAYRF | Bat SARS coronavirus HKU3-13 [742000] |
| FAMQMAYRF | Bat coronavirus BM48-31/BGR/2008 [864596] |
| FAMQMAYRF | SARS coronavirus MA15 ExoN1 [633140] |
| FAMQMAYRF | SARS coronavirus MA15 ExoN1 [633140] |
| FAMQMAYRF | SARS coronavirus ExoN1 [627440] |
| FAMQMAYRF | Human SARS coronavirus (SARS-CoV) [694009] |
| FAMQMAYRF | Human SARS coronavirus (SARS-CoV) [694009] |
| FAMQMAYRF | Human SARS coronavirus (SARS-CoV) [694009] |
| FAMQMAYRF | Bat coronavirus 279/2005 (BtCoV) (BtCoV/279/2005) [389167] |
| FAMQMAYRF | Human SARS coronavirus (SARS-CoV) [694009] |
| FAMQMAYRF | SARS coronavirus BJ202 [321149] |
| FAMQMAYRF | SARS coronavirus BJ162 [321147] |
| FAMQMAYRF | SARS coronavirus Frankfurt 1 [229992] |
| FAMQMAYRF | Human SARS coronavirus (SARS-CoV) [694009] |
| FAMQMAYRF | Human SARS coronavirus (SARS-CoV) [694009] |
| FAMQMAYRF | Human SARS coronavirus (SARS-CoV) [694009] |
| FAMQMAYRF | Human SARS coronavirus (SARS-CoV) [694009] |
| FAMQMAYRF | Human SARS coronavirus (SARS-CoV) [694009] |
| FAMQMAYRF | Human SARS coronavirus (SARS-CoV) [694009] |
| FAMQMAYRF | Human SARS coronavirus (SARS-CoV) [694009] |
| FAMQMAYRF | Human SARS coronavirus (SARS-CoV) [694009] |
| FAMQMAYRF | Human SARS coronavirus (SARS-CoV) [694009] |
| FAMQMAYRF | Human SARS coronavirus (SARS-CoV) [694009] |
| FAMQMAYRF | Human SARS coronavirus (SARS-CoV) [694009] |
| FAMQMAYRF | Human SARS coronavirus (SARS-CoV) [694009] |
| FAMQMAYRF | SARS coronavirus ZJ02 [353145] |
| FAMQMAYRF | Bat coronavirus Rp3/2004 (BtCoV/Rp3/2004) (SARS-like coronavirus Rp3) [349344] |
| FAMQMAYRF | Bat SARS coronavirus HKU3-3 [338606] |
| FAMQMAYRF | Bat SARS coronavirus HKU3-2 [338605] |
| FAMQMAYRF | Bat coronavirus HKU3 (BtCoV) (SARS-like coronavirus HKU3) [442736] |
| FAMQMAYRF | SARS coronavirus ZJ0301 [344702] |
| FAMQMAYRF | SARS coronavirus civet020 [285949] |
| FAMQMAYRF | SARS coronavirus civet019 [285948] |
| FAMQMAYRF | SARS coronavirus civet014 [285947] |
| FAMQMAYRF | SARS coronavirus civet010 [285946] |
| FAMQMAYRF | SARS coronavirus A022 [304858] |
| FAMQMAYRF | SARS coronavirus C029 [305418] |
| FAMQMAYRF | SARS coronavirus C028 [305417] |
| FAMQMAYRF | SARS coronavirus C025 [305416] |
| FAMQMAYRF | SARS coronavirus C019 [305415] |
| FAMQMAYRF | SARS coronavirus C018 [305414] |
| FAMQMAYRF | SARS coronavirus C017 [305413] |
| FAMQMAYRF | SARS coronavirus C014 [305412] |
| FAMQMAYRF | SARS coronavirus C013 [305411] |
| FAMQMAYRF | SARS coronavirus B040 [305410] |
| FAMQMAYRF | SARS coronavirus B039 [299335] |
| FAMQMAYRF | SARS coronavirus B033 [305409] |
| FAMQMAYRF | SARS coronavirus B029 [305408] |
| FAMQMAYRF | SARS coronavirus B024 [305407] |
| FAMQMAYRF | SARS coronavirus B012 [305406] |
| FAMQMAYRF | SARS coronavirus A031 [305405] |
| FAMQMAYRF | SARS coronavirus A030 [305404] |
| FAMQMAYRF | SARS coronavirus A021 [305403] |
| FAMQMAYRF | SARS coronavirus A013 [305402] |
| FAMQMAYRF | SARS coronavirus A001 [305401] |
| FAMQMAYRF | SARS coronavirus WH20 [312027] |
| FAMQMAYRF | SARS coronavirus PC4-241 [296841] |
| FAMQMAYRF | SARS coronavirus PC4-199 [296840] |
| FAMQMAYRF | SARS coronavirus PC4-145 [296839] |
| FAMQMAYRF | SARS coronavirus PC4-137 [296838] |
| FAMQMAYRF | SARS coronavirus PC4-115 [296837] |
| FAMQMAYRF | SARS coronavirus GZ0403 [293321] |
| FAMQMAYRF | SARS coronavirus PC4-205 [293320] |
| FAMQMAYRF | SARS coronavirus PC4-127 [293319] |
| FAMQMAYRF | SARS Coronavirus CDC#200301157 [292360] |
| FAMQMAYRF | SARS coronavirus GD322 [291613] |
| FAMQMAYRF | SARS coronavirus TJF [284672] |
| FAMQMAYRF | SARS coronavirus HHS-2004 [285267] |
| FAMQMAYRF | SARS coronavirus LLJ-2004 [273522] |
| FAMQMAYRF | SARS coronavirus Sino3-11 [255729] |
| FAMQMAYRF | SARS coronavirus Sino1-11 [255730] |
| FAMQMAYRF | SARS coronavirus NS-1 [260743] |
| FAMQMAYRF | SARS coronavirus TW9 [258972] |
| FAMQMAYRF | SARS coronavirus TW8 [258971] |
| FAMQMAYRF | SARS coronavirus TW7 [258970] |
| FAMQMAYRF | SARS coronavirus TW6 [258969] |
| FAMQMAYRF | SARS coronavirus TW5 [258968] |
| FAMQMAYRF | SARS coronavirus TW4 [258967] |
| FAMQMAYRF | SARS coronavirus TW3 [258966] |
| FAMQMAYRF | SARS coronavirus TW2 [258965] |
| FAMQMAYRF | SARS coronavirus TW11 [258964] |
| FAMQMAYRF | SARS coronavirus TW10 [258963] |
| FAMQMAYRF | SARS coronavirus ShanghaiQXC2 [258508] |
| FAMQMAYRF | SARS coronavirus BJ302 [252654] |
| FAMQMAYRF | SARS coronavirus BJ302 [252654] |
| FAMQMAYRF | SARS coronavirus BJ302 [252654] |
| FAMQMAYRF | SARS coronavirus BJ302 [252654] |
| FAMQMAYRF | SARS coronavirus BJ302 [252654] |
| FAMQMAYRF | SARS coronavirus BJ302 [252654] |
| FAMQMAYRF | SARS coronavirus BJ302 [252654] |
| FAMQMAYRF | SARS coronavirus BJ302 [252654] |
| FAMQMAYRF | SARS coronavirus GZ02 [256753] |
| FAMQMAYRF | SARS coronavirus PUMC03 [253435] |
| FAMQMAYRF | SARS coronavirus PUMC02 [253434] |
| FAMQMAYRF | SARS coronavirus PUMC01 [253433] |
| FAMQMAYRF | SARS coronavirus CUHK-AG03 [239243] |
| FAMQMAYRF | SARS coronavirus CUHK-AG02 [239242] |
| FAMQMAYRF | SARS coronavirus CUHK-AG01 [239241] |
| FAMQMAYRF | SARS coronavirus ZJ01 [230471] |
| FAMQMAYRF | Bat coronavirus Rp/Shaanxi2011 [1283332] |
| FAMQMAYRF | Bat coronavirus Cp/Yunnan2011 [1283333] |
| FAMQMAYRF | SARS coronavirus ExoN1 [627440] |
| FAMQMAYRF | Bat SARS-like coronavirus Rs3367 [1415834] |
| FAMQMAYRF | Bat SARS-like coronavirus WIV1 [1415852] |
| FAMQMAYRF | Bat SARS-like coronavirus RsSHC014 [1415851] |
| FKCYGVS | Rhinolophus affinis coronavirus [1487703] |
| FKCYGVS | Rhinolophus affinis coronavirus [1487703] |
| FKCYGVS | Bat SARS-like coronavirus [1508227] |
| FKCYGVS | SARS-like coronavirus WIV16 [1739625] |
| FKCYGVS | Bat coronavirus [1508220] |
| FKCYGVS | Bat coronavirus [1508220] |
| FKCYGVS | Human SARS coronavirus (SARS-CoV) [694009] |
| FKCYGVS | Bat SARS-like coronavirus [1508227] |
| FKCYGVS | Bat SARS-like coronavirus [1508227] |
| FKCYGVS | Bat SARS-like coronavirus [1508227] |
| FKCYGVS | Bat SARS-like coronavirus [1508227] |
| FKCYGVS | Bat SARS-like coronavirus [1508227] |
| FKCYGVS | Bat SARS-like coronavirus [1508227] |
| FKCYGVS | Bat SARS-like coronavirus [1508227] |
| FKCYGVS | Bat SARS-like coronavirus [1508227] |
| FKCYGVS | Bat SARS-like coronavirus [1508227] |
| FKCYGVS | Bat SARS-like coronavirus [1508227] |
| FKCYGVS | Bat SARS-like coronavirus [1508227] |
| FKCYGVS | Bat SARS-like coronavirus [1508227] |
| FKCYGVS | Bat SARS-like coronavirus [1508227] |
| FKCYGVS | SARS coronavirus Urbani [228330] |
| FKCYGVS | SARS coronavirus Urbani [228330] |
| FKCYGVS | Human SARS coronavirus (SARS-CoV) [694009] |
| FKCYGVS | Coronavirus BtRI-BetaCoV/SC2018 [2591233] |
| FKCYGVS | Coronavirus BtRs-BetaCoV/YN2018B [2591235] |
| FKCYGVS | Coronavirus BtRs-BetaCoV/YN2018D [2591237] |
| FKCYGVS | Coronavirus BtRs-BetaCoV/YN2018A [2591234] |
| FKCYGVS | Coronavirus BtRs-BetaCoV/YN2018C [2591236] |
| FKCYGVS | SARS coronavirus Frankfurt1-v01 [391355] |
| FKCYGVS | SARS coronavirus WF188 [385687] |
| FKCYGVS | SARS coronavirus ES191 [385685] |
| FKCYGVS | SARS coronavirus ES260 [385686] |
| FKCYGVS | SARS coronavirus CS21 [385683] |
| FKCYGVS | SARS coronavirus CS24 [385684] |
| FKCYGVS | Human SARS coronavirus (SARS-CoV) [694009] |
| FKCYGVS | SARS coronavirus BJ182a [511432] |
| FKCYGVS | SARS coronavirus BJ182b [511433] |
| FKCYGVS | SARS coronavirus BJ182-4 [511430] |
| FKCYGVS | SARS coronavirus BJ182-8 [511431] |
| FKCYGVS | SARS coronavirus BJ182-12 [511429] |
| FKCYGVS | SARS coronavirus P2 [627442] |
| FKCYGVS | SARS coronavirus Rs_672/2006 [722424] |
| FKCYGVS | SARS coronavirus ExoN1 [627440] |
| FKCYGVS | SARS coronavirus wtic-MB [698419] |
| FKCYGVS | SARS coronavirus ExoN1 [627440] |
| FKCYGVS | SARS coronavirus ExoN1 [627440] |
| FKCYGVS | SARS coronavirus ExoN1 [627440] |
| FKCYGVS | SARS coronavirus ExoN1 [627440] |
| FKCYGVS | SARS coronavirus MA15 ExoN1 [633140] |
| FKCYGVS | SARS coronavirus MA15 ExoN1 [633140] |
| FKCYGVS | SARS coronavirus ExoN1 [627440] |
| FKCYGVS | SARS coronavirus MA15 [633137] |
| FKCYGVS | SARS coronavirus wtic-MB [698419] |
| FKCYGVS | SARS coronavirus ExoN1 [627440] |
| FKCYGVS | SARS coronavirus MA15 ExoN1 [633140] |
| FKCYGVS | SARS coronavirus ExoN1 [627440] |
| FKCYGVS | SARS coronavirus ExoN1 [627440] |
| FKCYGVS | SARS coronavirus HKU-39849 [228404] |
| FKCYGVS | SARS coronavirus HKU-39849 [228404] |
| FKCYGVS | Bat SARS coronavirus HKU3-4 [742001] |
| FKCYGVS | Bat SARS coronavirus HKU3-5 [742002] |
| FKCYGVS | Bat SARS coronavirus HKU3-6 [742003] |
| FKCYGVS | Bat SARS coronavirus HKU3-7 [742004] |
| FKCYGVS | Bat SARS coronavirus HKU3-8 [742005] |
| FKCYGVS | Bat SARS coronavirus HKU3-9 [742006] |
| FKCYGVS | Bat SARS coronavirus HKU3-10 [741997] |
| FKCYGVS | Bat SARS coronavirus HKU3-11 [741998] |
| FKCYGVS | Bat SARS coronavirus HKU3-12 [741999] |
| FKCYGVS | Bat SARS coronavirus HKU3-13 [742000] |
| FKCYGVS | SARS coronavirus MA15 ExoN1 [633140] |
| FKCYGVS | SARS coronavirus MA15 ExoN1 [633140] |
| FKCYGVS | SARS coronavirus ExoN1 [627440] |
| FKCYGVS | Human SARS coronavirus (SARS-CoV) [694009] |
| FKCYGVS | Human SARS coronavirus (SARS-CoV) [694009] |
| FKCYGVS | Human SARS coronavirus (SARS-CoV) [694009] |
| FKCYGVS | Bat coronavirus 279/2005 (BtCoV) (BtCoV/279/2005) [389167] |
| FKCYGVS | Human SARS coronavirus (SARS-CoV) [694009] |
| FKCYGVS | SARS coronavirus BJ202 [321149] |
| FKCYGVS | SARS coronavirus BJ162 [321147] |
| FKCYGVS | SARS coronavirus GD322 [291613] |
| FKCYGVS | SARS coronavirus Frankfurt 1 [229992] |
| FKCYGVS | Human SARS coronavirus (SARS-CoV) [694009] |
| FKCYGVS | Human SARS coronavirus (SARS-CoV) [694009] |
| FKCYGVS | Human SARS coronavirus (SARS-CoV) [694009] |
| FKCYGVS | Human SARS coronavirus (SARS-CoV) [694009] |
| FKCYGVS | Human SARS coronavirus (SARS-CoV) [694009] |
| FKCYGVS | Human SARS coronavirus (SARS-CoV) [694009] |
| FKCYGVS | Human SARS coronavirus (SARS-CoV) [694009] |
| FKCYGVS | Human SARS coronavirus (SARS-CoV) [694009] |
| FKCYGVS | Human SARS coronavirus (SARS-CoV) [694009] |
| FKCYGVS | Human SARS coronavirus (SARS-CoV) [694009] |
| FKCYGVS | Human SARS coronavirus (SARS-CoV) [694009] |
| FKCYGVS | Human SARS coronavirus (SARS-CoV) [694009] |
| FKCYGVS | SARS coronavirus ZJ02 [353145] |
| FKCYGVS | Bat coronavirus Rp3/2004 (BtCoV/Rp3/2004) (SARS-like coronavirus Rp3) [349344] |
| FKCYGVS | Bat SARS coronavirus HKU3-3 [338606] |
| FKCYGVS | Bat SARS coronavirus HKU3-2 [338605] |
| FKCYGVS | Bat coronavirus HKU3 (BtCoV) (SARS-like coronavirus HKU3) [442736] |
| FKCYGVS | SARS coronavirus ZJ0301 [344702] |
| FKCYGVS | SARS coronavirus civet020 [285949] |
| FKCYGVS | SARS coronavirus civet019 [285948] |
| FKCYGVS | SARS coronavirus civet014 [285947] |
| FKCYGVS | SARS coronavirus civet010 [285946] |
| FKCYGVS | SARS coronavirus A022 [304858] |
| FKCYGVS | SARS coronavirus C029 [305418] |
| FKCYGVS | SARS coronavirus C028 [305417] |
| FKCYGVS | SARS coronavirus C025 [305416] |
| FKCYGVS | SARS coronavirus C019 [305415] |
| FKCYGVS | SARS coronavirus C018 [305414] |
| FKCYGVS | SARS coronavirus C017 [305413] |
| FKCYGVS | SARS coronavirus C014 [305412] |
| FKCYGVS | SARS coronavirus C013 [305411] |
| FKCYGVS | SARS coronavirus B040 [305410] |
| FKCYGVS | SARS coronavirus B039 [299335] |
| FKCYGVS | SARS coronavirus B033 [305409] |
| FKCYGVS | SARS coronavirus B029 [305408] |
| FKCYGVS | SARS coronavirus B024 [305407] |
| FKCYGVS | SARS coronavirus B012 [305406] |
| FKCYGVS | SARS coronavirus A031 [305405] |
| FKCYGVS | SARS coronavirus A030 [305404] |
| FKCYGVS | SARS coronavirus A021 [305403] |
| FKCYGVS | SARS coronavirus A013 [305402] |
| FKCYGVS | SARS coronavirus A001 [305401] |
| FKCYGVS | SARS coronavirus WH20 [312027] |
| FKCYGVS | SARS coronavirus PC4-241 [296841] |
| FKCYGVS | SARS coronavirus PC4-199 [296840] |
| FKCYGVS | SARS coronavirus PC4-145 [296839] |
| FKCYGVS | SARS coronavirus PC4-137 [296838] |
| FKCYGVS | SARS coronavirus PC4-115 [296837] |
| FKCYGVS | SARS coronavirus PC4-205 [293320] |
| FKCYGVS | SARS coronavirus PC4-127 [293319] |
| FKCYGVS | SARS Coronavirus CDC#200301157 [292360] |
| FKCYGVS | SARS coronavirus TJF [284672] |
| FKCYGVS | SARS coronavirus HHS-2004 [285267] |
| FKCYGVS | SARS coronavirus LLJ-2004 [273522] |
| FKCYGVS | SARS coronavirus Sino3-11 [255729] |
| FKCYGVS | SARS coronavirus Sino1-11 [255730] |
| FKCYGVS | SARS coronavirus sf098 [264990] |
| FKCYGVS | SARS coronavirus cw037 [265124] |
| FKCYGVS | SARS coronavirus cw049 [264989] |
| FKCYGVS | SARS coronavirus NS-1 [260743] |
| FKCYGVS | SARS coronavirus TW9 [258972] |
| FKCYGVS | SARS coronavirus TW8 [258971] |
| FKCYGVS | SARS coronavirus TW7 [258970] |
| FKCYGVS | SARS coronavirus TW6 [258969] |
| FKCYGVS | SARS coronavirus TW5 [258968] |
| FKCYGVS | SARS coronavirus TW4 [258967] |
| FKCYGVS | SARS coronavirus TW3 [258966] |
| FKCYGVS | SARS coronavirus TW2 [258965] |
| FKCYGVS | SARS coronavirus TW11 [258964] |
| FKCYGVS | SARS coronavirus TW10 [258963] |
| FKCYGVS | SARS coronavirus ShanghaiQXC2 [258508] |
| FKCYGVS | SARS coronavirus TW-PH2 [264388] |
| FKCYGVS | SARS coronavirus TW-PH1 [264387] |
| FKCYGVS | SARS coronavirus TW-YM4 [264386] |
| FKCYGVS | SARS coronavirus TW-YM3 [264385] |
| FKCYGVS | SARS coronavirus TW-YM2 [264384] |
| FKCYGVS | SARS coronavirus TW-YM1 [264383] |
| FKCYGVS | SARS coronavirus TW-GD5 [264382] |
| FKCYGVS | SARS coronavirus TW-GD4 [264381] |
| FKCYGVS | SARS coronavirus TW-GD3 [264380] |
| FKCYGVS | SARS coronavirus TW-GD2 [264379] |
| FKCYGVS | SARS coronavirus TW-GD1 [264378] |
| FKCYGVS | SARS coronavirus TW-KC3 [264377] |
| FKCYGVS | SARS coronavirus TW-KC1 [264376] |
| FKCYGVS | SARS coronavirus TW-JC2 [264375] |
| FKCYGVS | SARS coronavirus TW-HP4 [264374] |
| FKCYGVS | SARS coronavirus TW-HP3 [264373] |
| FKCYGVS | SARS coronavirus TW-HP2 [264372] |
| FKCYGVS | SARS coronavirus TW-HP1 [264371] |
| FKCYGVS | SARS coronavirus CUHK-L2 [260550] |
| FKCYGVS | SARS coronavirus BJ302 [252654] |
| FKCYGVS | SARS coronavirus BJ302 [252654] |
| FKCYGVS | SARS coronavirus BJ302 [252654] |
| FKCYGVS | SARS coronavirus BJ302 [252654] |
| FKCYGVS | SARS coronavirus BJ302 [252654] |
| FKCYGVS | SARS coronavirus BJ302 [252654] |
| FKCYGVS | SARS coronavirus BJ302 [252654] |
| FKCYGVS | SARS coronavirus BJ302 [252654] |
| FKCYGVS | SARS coronavirus GZ02 [256753] |
| FKCYGVS | SARS coronavirus PUMC03 [253435] |
| FKCYGVS | SARS coronavirus PUMC02 [253434] |
| FKCYGVS | SARS coronavirus PUMC01 [253433] |
| FKCYGVS | SARS coronavirus CUHK-AG03 [239243] |
| FKCYGVS | SARS coronavirus CUHK-AG02 [239242] |
| FKCYGVS | SARS coronavirus CUHK-AG01 [239241] |
| FKCYGVS | SARS coronavirus ZJ01 [230471] |
| FKCYGVS | Bat coronavirus Rp/Shaanxi2011 [1283332] |
| FKCYGVS | Bat coronavirus Cp/Yunnan2011 [1283333] |
| FKCYGVS | SARS coronavirus ExoN1 [627440] |
| FKCYGVS | Bat SARS-like coronavirus Rs3262-2 [1415831] |
| FKCYGVS | Bat SARS-like coronavirus Rs3267-2 [1415833] |
| FKCYGVS | Bat SARS-like coronavirus Rs3262-1 [1415830] |
| FKCYGVS | Bat SARS-like coronavirus Rs4081 [1415839] |
| FKCYGVS | Bat SARS-like coronavirus Rs3367 [1415834] |
| FKCYGVS | Bat SARS-like coronavirus Rs4092 [1415845] |
| FKCYGVS | Bat SARS-like coronavirus WIV1 [1415852] |
| FKCYGVS | Bat SARS-like coronavirus Rs4105 [1415848] |
| FKCYGVS | Bat SARS-like coronavirus Rs4096 [1415846] |
| FKCYGVS | Bat SARS-like coronavirus Rs4080 [1415838] |
| FKCYGVS | Bat SARS-like coronavirus Rs4079 [1415837] |
| FKCYGVS | Bat SARS-like coronavirus Rs4075 [1415836] |
| FKCYGVS | Bat SARS-like coronavirus Rs4097 [1415847] |
| FKCYGVS | Bat SARS-like coronavirus Rs4087-2 [1415843] |
| FKCYGVS | Bat SARS-like coronavirus Rs3369 [1415835] |
| FKCYGVS | Bat SARS-like coronavirus Rs4110 [1415850] |
| FKCYGVS | Bat SARS-like coronavirus Rs4084 [1415840] |
| FKCYGVS | Bat SARS-like coronavirus Rs4108 [1415849] |
| FKCYGVS | Bat SARS-like coronavirus Rs3267-1 [1415832] |
| FKCYGVS | Bat SARS-like coronavirus Rs4085 [1415841] |
| FKCYGVS | Bat SARS-like coronavirus Rs4090 [1415844] |
| FKCYGVS | Bat SARS-like coronavirus Rs4087-1 [1415842] |
| FKCYGVS | Bat SARS-like coronavirus RsSHC014 [1415851] |
| IKWPWYI | Bat Hp-betacoronavirus/Zhejiang2013 [1541205] |
| IKWPWYI | Bat coronavirus [1508220] |
| IKWPWYI | Canada goose coronavirus [2569586] |
| IKWPWYI | Bat coronavirus HKU9-3 [424369] |
| IKWPWYI | Bat coronavirus HKU9-5-1 [875614] |
| IKWPWYI | Bat coronavirus HKU9-5-2 [875615] |
| IKWPWYI | Bat coronavirus HKU9-10-1 [875612] |
| IKWPWYI | Bat coronavirus HKU9-10-2 [875613] |
| IKWPWYI | Rousettus bat coronavirus/Kenya/KY06/2006 [983925] |
| IKWPWYI | Sparrow coronavirus HKU17 [1159906] |
| IKWPWYI | Common moorhen coronavirus HKU21 [1159902] |

## Claims

1. A peptide comprising a sequence motif of any of SEQ ID NO: 1-74, comprising at most 30 consecutive amino acids occurring in a spike protein of a coronavirus and comprising any of the recited sequence motifs.

2. The peptide of claim 1, consisting of the sequence motif of any of SEQ ID NO: 1-74 or consisting of a combination of sequence motifs of any of SEQ ID NO: 1-74, optionally, consisting of the sequence motif of any of SEQ ID NO: 1-74.

3. The peptide of any of the preceding claims comprising at least one of the sequence motifs of SEQ ID NO: 1, 2 or 3,
wherein the peptide optionally comprises a plurality of peptides of any of claims 1 or 2, wherein the peptide preferably comprises at least two of the sequence motifs of SEQ ID NO: 1, 2 or 3.

4. The peptide of any of the preceding claims comprising SEQ ID NO: 1.

5. The peptide of any of the preceding claims comprising SEQ ID NO: 2.

6. The peptide of any of the preceding claims comprising SEQ ID NO: 3, wherein the peptide optionally comprises SEQ Id NO: 1, 2 and 3.

7. The peptide of any of the preceding claims, wherein the peptide is conjugated to a carrier protein, preferably, a carrier protein derived from an infectious agent selected from the group comprising influenza hemagglutinin, diphteria toxoid, pertussis toxoid, tetanus toxoid, and hepatitis B virus surface antigen, wherein the peptide optionally is a fusion peptide.

8. A nucleic acid encoding the peptide of any of the preceding claims, wherein the nucleic acid is selected from the group comprising an expression vector or a virus.

9. A composition comprising the peptide of any of claims 1-7 or the nucleic acid of claim 8, wherein the composition does not comprise an adjuvant.

10. A composition comprising the peptide of any of claims 1-7 or the nucleic acid of claim 8, wherein the composition further comprises at least one adjuvant.

11. A composition of any of claims 9 or 10 comprising the peptide of any of claims 1-7, wherein the composition comprises peptides consisting of SEQ ID NO: 1, 2 and 3.

12. A pharmaceutical composition comprising the peptide of any of claims 1-7 or the nucleic acid of claim 8 or the composition of any of claims 9-11, wherein the composition preferably is a vaccine.

13. The peptide of any of claims 1-7, the nucleic acid of claim 8 or the pharmaceutical composition of claim 12 for use in vaccination against at least one coronavirus, preferably, against SARS-CoV-2.

14. The pharmaceutical composition for use of claim 13, wherein the composition is for use in vaccination against at least SARS-CoV-2 and SARS-CoV.

15. The pharmaceutical composition for use of any of claims 13 or 14, wherein the composition further comprises or encodes an influenza antigen and is for use in vaccination against a coronavirus and influenza.
